# EUROPEAN PATENT SPECIFICATION

(11) **EP 0 665 834 B1**
(45) Date of publication and mention of the grant of the patent: **30.05.2001**
(21) Application number: 93922605.6
(22) Date of filing: 14.10.1993
(51) Int. Cl.: C07D 215/20, A61K 31/435, C07D 405/04, C07D 219/06, C07D 215/54

(54) **QUINOLONE AND ACRIDINONE DERIVATIVES FOR THE TREATMENT OF URINARY INCONTINENCE**
CHINOLON UND ACRIDINON DERIVATE ZUR BEHANDLUNG DER HARNINKONTINENZ
DERIVES DE QUINOLONE ET D'ACRIDINONE UTILISES DANS LE TRAITEMENT DE L'INCONTINENCE URINAIRE

(30) Priority: 20.10.1992 GB 9221989; 20.04.1993 GB 9308065
(43) Date of publication of application: 09.08.1995
(73) Proprietor: AstraZeneca AB, 151 85 Södertälje (SE)
(72) Inventor: OHNMACHT, Cyrus, John, Wilmington, DE 19810 (US); TRAINOR, Diane, Amy, Wilmington, DE 19803 (US); FORST, Janet, Marie, Wilmington, DE 19808 (US); STEIN, Mark, Morris, Wilmington, DE 19803 (US); HARRIS, Robert, Joseph, Claymont, DE 19703 (US)
(74) Representative: Bill, Kevin
(86) International application number: GB9302125
(87) International publication number: WO9408966

(56) References cited:
- EP-A- 0 539 153
- EP-A- 0 539 154
- DE-A- 2 003 148
- DE-A- 2 018 738

## Description

This invention relates to a group of compounds which are useful in the treatment of bladder instability in mammals such as man. More specifically, this invention relates to a group of 4,6,7,8-tetrahydro-5(1H)-quinolones, their use in the treatment of urinary incontinence in mammals (including man), processes for preparing them and pharmaceutical compositions containing them.

The existing treatments for urinary incontinence are generally poor, relying on drugs that had originally been developed for other indications. One group of such drugs comprises the calcium channel blockers, such as nifedipine, which were originally developed and are primarily used as cardiovascular agents.

Nifedipine belongs to a structural class of compounds known as the dihydropyridines. This structural class has been extensively investigated, and the structural requirements for calcium blocking activity are now quite well established. Thus, as described on Chapter 14.1 of the medicinal chemistry text book, Comprehensive Medicinal Chemistry, Volume 3, Edited by John C. Emmett and Published by Pergamon Press in 1990, the compounds possess a 1,4-dihydropyridine ring having, optimally, an aryl group at the 4-position and ester groups at the 3- and 5-positions. Removing the ester groups or replacing them with acetyl or cyano groups is associated with a reduction in activity. Generally, the compounds have methyl groups at the 2- and 6-positions.

Grinshteins et al, Khim. Geterotsikl. Soedin. (6), 1118-20, 1967 disclose the compounds 3-cyano-4-phenyl-2,7,7-trimethyl-4,6,7,8-tetrahydro-5(1H)-quinolone and 3-ethanoyl-4-phenyl-2,7,7-trimethyl-4,6,7,8-tetrahydro-5(1H)-quinolone. Vitolinya et al, Khim.-Farm. Zh., 15(1), 39-42, 1981 discloses an investigation of the effect of several 4,6,7,8-tetrahydro-5(1H)-quinolones having an ester or cyano group at the 3-position on the cardiovascular system and on intestinal smooth muscle. 3-Cyano-4-phenyl-2,7,7-trimethyl-4,6,7,8-tetrahydro-5(1H)-quinolone is reported to possess hypotensive properties and to be capable of blocking the spasmogenic effect of both acetylcholine and barium chloride on intestinal smooth muscle.

DE 2003148 discloses a group of 1,4-dihydropyridine derivatives, including certain 4,6,7,8-tetrahydro-5(1H)-quinolones, which possess an ester or keto group at the 3-position and which are said to display a wide and multi-faceted pharmacological spectrum of action. The main effects said to be displayed by the compounds include strong muscular spasmloytic effects which become evident in the smooth musculature of the gastrointestinal tract, of the urogenital tract and of the respiratory system. Other main effects are stated to be on the heart (a "heart-relieving" effect) and in reducing the blood pressure of normotonic and hypertonic animals, so that they can be used as antihypertensive agents.

S. H. Jain et al, Indian Journal of Chemistry, Volume 30B, November, 1991, pages 1037-1040 discloses the synthesis and pharmacological screening of certain 9-(substituted phenyl)-1,8--(2H,5H)-acridinediones. The compounds were found to possess varying degrees of hypotensive, anti-inflammatory and anti-implantation activities.

It is known that bladder tissue is excitable and that urinary incontinence can be caused by uncontrolled or unstable bladder contractions. A group of compounds have been found that are unexpectedly capable of relaxing bladder smooth muscle, thus preventing or ameliorating uncontrolled or unstable bladder contractions. Hence, the compounds may be useful for the treatment of urge incontinence, which includes for example detrusor instability, which may result from cystitis, urethritis, tumors, stones, diverticuli or outflow obstruction; and detrusor hyperreflexia, which may result from stroke, dementia, parkinsons, suprasacral spinalcord injury or suprasacral spinalcord disease. Some of the compounds have been found to possess the further unexpected property that they are capable of acting selectively on the bladder without at the same time significantly affecting the cardiovascular system, as indicated by heart rate and blood pressure measurements. Thus, these compounds may be particularly useful to treat urinary incontinence in patients, such as for example the elderly, for whom cardiovascular effects, such as a hypotensive effect, are particularly undesirable.

It has also unexpectedly been found that compounds according to the invention are potassium channel openers. It is known that by functioning to open potassium channels, potassium channel opening compounds can thereby function to relax smooth muscle. While not wishing to be bound by theory, it is accordingly believed that the compounds of this invention function by opening potassium channels in bladder cells and thereby relax bladder smooth muscle tissue, thus preventing or ameliorating uncontrolled bladder contractions which can cause urinary incontinence. Nurse D. A., Restorick J.H., and Mundy A.R., British Journal of Urology, (1991), 68, 27-31 discloses that cromakalim, which is well known as a potassium channel opener, has been found to be effective in a preliminary clinical trial for the treatment of urinary incontinence.

The structural requirements for activity in the 4,6,7,8-tetrahydro-5(1H)-quinolones according to the present invention have been found to be different from those expected of dihydropyridine calcium blockers. It is accordingly believed that the present invention is based upon the discovery of a new pharmacological class of bladder-relaxant dihydropyridines.

This invention provides a compound of formula I (formula set out, together with other formulae referred to by Roman numerals, on pages following the Examples), or a pharmaceutically acceptable salt thereof, wherein: either
R² is hydrogen, (1-6C)alkyl or (1-4C)fluoroalkyl; and
R³ is hydrogen, cyano, (1-6C)alkyl, (1-6C)fluoroalkyl or ethanoyl; or
R² and R³ when taken together form a 1,4-butandiyl;
R⁴ is 2- or 3-thienyl or furyl substituted at the 4- and/or 5-position(s) by a radical or radicals independently selected from a group (a) consisting of nitro, cyano, halo, (1-4C)alkyl, (1-4C)alkylsulphonyl and 2-thienyl provided that a 3-thienyl or furyl group may only be substituted at the 5-position; or
R⁴ is a 2-pyridyl which is substituted at the 5 position and/or either at the 4 position or the 6 position by a member of the above group (a); or
R⁴ is a 3-pyridyl which is substituted at the 6 position by a member of the above group (a); or
R⁴ is a 4-pyridyl which is substituted at the 2 position by a member of the above group (a); or
R⁴ is a group of formula II, wherein:
   R⁷ is hydrogen; and
   R⁸ and R⁹ are independently selected from hydrogen, hydroxy (1-4C)alkoxy, nitro, cyano, (1-4C)fluoroalkyl, (1-4C)fluoroalkoxy, halo, (1-4C)alkyl, (1-4C)alkanoyl, phenyl and (1-4C)alkylsulphonyl; or
   R⁸ and R⁹ taken together are (1-3C)alkylenedioxy; and
   R¹⁰ and R¹¹ are each independently hydrogen or (1-4C)alkyl, but excluding 3-cyano-4-phenyl-2,7,7-trimethyl-4,6,7,8-tetrahydro--5(1H)-quinolone and 3-ethanoyl-4-phenyl-2,7,7-trimethyl-4,6,7,8-tetrahydro-5(1H)-quinolone.

In this specification the terms "alkyl" and "alkoxy" include both straight and branched chain radicals, but it is to be understood that references to individual radicals such as "propyl" or "propoxy" embrace only the straight chain ("normal") radical, branched chain isomers such as "isopropyl" or "isopropoxy" being referred to specifically.

The term "halo" is inclusive of fluoro, chloro, bromo, and iodo unless noted otherwise.

Particular values of 2- or 3-thienyl or furyl substituted at the 4- and/or 5-positions include 4-bromo-2-thienyl, 5-bromo-2-thienyl, 5-methylsulphonyl-2-thienyl, 5-methyl-2-thienyl, 5-(2-thienyl)-2-thienyl, 4-nitro-2-thienyl, 5-nitro-2-thienyl, 4-cyano-2-thienyl, and 5-nitro-3-thienyl.

Particular values of (1-6C)alkyl include methyl, ethyl, propyl, isopropyl, butyl, isobutyl, sec-butyl, and tert-butyl, pentyl, 3-methylbutyl, 1-ethylpropyl, hexyl, or 4-methylpentyl.

Particular values of (1-4C)alkyl include methyl, ethyl, propyl, isopropyl, butyl, isobutyl, sec-butyl, and tert-butyl.

Particular values of (1-4C)fluoroalkyl include trifluoromethyl and pentafluoroethyl.

Particular values of (1-4C)alkoxy include methoxy, ethoxy propoxy, isopropoxy, butoxy, isobutoxy, sec-butoxy, and tert-butoxy.

Particular values of (1-4C)fluoroalkoxy include trifluoromethoxy and pentafluoroethoxy.

A particular value of (1-4C)alkanoyl is ethanoyl.

A particular value of (1-4C)alkylsulphonyl is methanesulphonyl.

Particular values for (1-3C)alkylenedioxy are methylenedioxy and ethylenedioxy.

Preferred values of R⁸ include hydrogen, hydroxy, methoxy, nitro, cyano, trifluoromethyl, trifluoromethoxy, methyl, ethyl, isopropyl and halo and ethanoyl.

Preferred values of R⁹ include hydrogen, hydroxy, methoxy, nitro, cyano, trifluoromethyl, trifluoromethoxy, methyl, ethyl, isopropyl and halo.

Particular values for R² are methyl, ethyl, propyl, isopropyl, butyl, isobutyl, sec-butyl, tert-butyl and trifluoromethyl.

Particular values for R³ are hydrogen, cyano, ethanoyl, or together with R² 1,4-butandiyl.

Compounds in which R³ is hydrogen or R² and R³ when taken together form a 1,4-butandiyl have been found to possess particularly good potency and selectivity for the bladder and are therefore preferred.

Compounds in which R² is trifluoromethyl have been found to possess particularly good potency, selectivity for the bladder and stability, and are therefore particularly preferred.

R⁴ is preferably a group of formula II.

Particular values for R⁴ are phenyl, 3-methoxyphenyl, 3-nitrophenyl, 3-cyanophenyl, 3-trifluoromethylphenyl, 3-trifluoromethyl-4-cyanophenyl, 4-trifluoromethylphenyl, 3-trifluoromethoxyphenyl, 3-fluorophenyl, 3-chlorophenyl, 3-chloro-4-fluorophenyl, 3-bromophenyl, 4-fluorophenyl, 4-chlorophenyl, 3-bromo-4-fluorophenyl, 3,4-dichlorophenyl, 4-methylphenyl, 3,4-methylenedioxyphenyl and 4-nitro-2-thienyl. Most preferably R⁴ is 3-nitrophenyl or 3-cyanophenyl.

Preferred values for R¹⁰ and R¹¹ are hydrogen and methyl. Preferably R¹⁰ and R¹¹ both represent hydrogen or both represent methyl.

A particularly preferred group of compounds of formula I is that wherein either
R² is methyl, ethyl, propyl, isopropyl, butyl, isobutyl, sec-butyl, tert-butyl or trifluoromethyl; and
R³ is hydrogen; or
R² and R³ when taken together form a 1,4-butandiyl;
R⁴ is 3-nitrophenyl or 3-cyanophenyl; and
R¹⁰ and R¹¹ are both hydrogen.

Particularly preferred compounds are 2-trifluoromethyl-4-(3-nitrophenyl)-4,6,7,8-tetrahydro-5(1H)-quinolone and 2-trifluoromethyl-4-(3-cyanophenyl)-4,6,7,8-tetrahydro-5(1H)-quinolone. Both of these compounds have been found to possess surprisingly good potency in vitro and surprisingly good selectivity for the bladder in vivo. They have also been found to possess chemical stability superior to that of the corresponding compounds of formula I wherein R² is methyl.

It will be appreciated by those skilled in the art that the compounds of formula I contain an asymmetrically substituted carbon, and accordingly may exist in, and be isolated in, optically-active and racemic forms. Some compounds may exhibit polymorphism. It is to be understood that the present invention encompasses any racemic, optically-active, polymorphic or stereoisomeric form, or mixtures thereof, which form possesses properties useful in the treatment of urinary incontinence, it being well known in the art how to prepare optically-active forms (for example, by resolution of the racemic form, by synthesis from optically-active starting materials, by chiral synthesis, or by chromatographic separation using a chiral stationary phase) and how to determine efficacy for the treatment of urinary incontinence by the standard tests described hereinafter.

A compound of formula I can be made by processes which include processes known in the chemical arts for the production of structurally analogous compounds. Such processes for the manufacture of a 4,6,7,8-tetrahydro-5(1H)-quinolones of formula I as defined above are provided as further features of the invention and are illustrated by the following procedures in which the meanings of generic radicals are as given above unless otherwise qualified. Such a process can be effected, generally,
(a) for a compound of formula I wherein R³ is hydrogen, by decarboxylation of a corresponding carboxylic acid of formula III in which R¹² and R¹³ together represent a bond, or decarboxylation and dehydration of a carboxylic acid of formula III in which R¹² is hydrogen and R¹³ is hydroxy.
(b) by reacting an unsaturated ketone of formula IV with the appropriate 1,3-cyclohexanedione and ammonia or an ammonium salt.
(c) for a compound of formula I wherein R² and R³ when taken together form a 1,4-butandiyl, by reacting an acridinedione of formula VI with a reducing agent.
(d) for a compound of formula I wherein R² and R³ when taken together form a 1,4-butandiyl, by reacting a ketone of formula VII with the appropriate 1,3-cyclohexanedione and ammonia or an ammonium salt.
(e) by reacting an enedione of formula XII with enamine of formula XIII in which Z is an imino group in the presence of ammonia or an ammonium salt.
(f) for a compound of formula I where R³ is ethanoyl or cyano reacting a compound of formula XV in which R^{3a} is ethanoyl or cyano with the appropriate 1,3-cyclohexanedione and the appropriate aldehyde of formula R⁴CHO.

The decarboxylation described in (a) can conveniently be carried out at an elevated temperature for example in the range of from 50 to 250°C, preferably, in the range of from 90 to 120 °C. Suitable solvents for the reaction include alcohols, for example, methanol or ethanol; dimethylsulfoxide; aromatic hydrocarbons such as toluene, and ethers, such as for example 1,2-dimethoxyethane or diglyme. The reaction is conveniently performed in the presence of an acid, for example concentrated sulphuric acid or p-toluenesulphonic acid.

Reaction (b) can conveniently be carried out at a temperature for example in the range of from 0 to 150°C, preferably at an elevated temperature, for example in the range of from 50 to 120 °C. Suitable solvents for the reaction include alcohols, for example, methanol or ethanol; dimethylsulfoxide; ethers, such as 1,2-dimethoxyethane or diglyme; and carboxylic acids, for example acetic acid. A convenient ammonium salt is for example ammonium acetate.

Reaction (c) can conveniently be carried out at a temperature for example in the range from 20 to 80 °C, preferably at a temperature in the range of 50 to 80 °C. Suitable solvents include alcohols, for example ethanol, pyridine and mixtures thereof. Suitable reducing agents include for example sodium borohydride.

Reaction (d) can conveniently be carried out at a temperature for example in the range of from 0 to 150°C, preferably at an elevated temperature, for example in the range of from 50 to 120 °C. Suitable solvents for the reaction include alcohols, for example, methanol or ethanol; dimethylsulfoxide; ethers, such as 1,2-dimethoxyethane or diglyme; and carboxylic acids, for example acetic acid. A convenient ammonium salt is for example ammonium acetate

In reaction (e), Z may be, for example, a dialkylimino group such as dimethylimino, or a cyclic imino group such as pyrrolidino or piperidino.

The enamine starting material of formula XIII may conveniently be formed in situ by reaction of the appropriate ketone of formula R²COCH₂R³ with the appropriate amine.

The reaction is conveniently carried out at a temperature in the range of from 20 to 80°C, preferably from 50 to 80°C. Suitable solvents include alcohols such as ethanol.

Reaction (f) is conveniently performed at a temperature in the range of from 0 to 150°C, preferably from 50 to 120°C. Suitable solvents include alcohols such as ethanol.

If not commercially available, the necessary starting materials for the processes such as those described following may be made by procedures which are selected from standard organic chemical techniques, techniques which are analogous to the synthesis of known, structurally similar compounds, or techniques which are analogous to the above described procedure or the procedures described in the examples.

An intermediate of formula III can be prepared by reacting an acetoacetic ester of formula X in which ORa is an alcohol residue such as for example 2-cyanoethoxy or ethoxy with an aldehyde of formula R⁴CHO and the appropriate 1,3-cyclohexanedione to give an ester of formula VIII as shown in scheme I. Hydrolysis of the ester, for example by treatment with aqueous sodium hydroxide in 1,2-dimethoxyethane yields an acid of formula III. In some cases an ester of formula VIIIa may be obtained instead of an ester of formula VIII, and this may be hydrolysed to give a compound of formula IIIa. Such a procedure is illustrated in Example 15 hereinafter.

An intermediate of formula IV may be prepared by reacting the corresponding benzaldehyde of formula R⁴CHO with a ketone of formula XIV. The reaction is conveniently preferred in the presence of a base, for example sodium hydroxide.

An intermediate acridinedione of formula VI can be prepared as shown in Scheme II, by reacting a corresponding aldehyde of formula R⁴CHO, or an acetal or hemiacetal thereof, with ammonia or an ammonium salt (such as ammonium acetate) and the appropriate 1,3-cyclohexanedione. The reaction can be carried out at a temperature in the range of from 0 to 150°C, preferably at an elevated temperature, for example in the range of from 50 to 120 °C. Suitable solvents for the reaction include alcohols, for example, methanol or ethanol; dimethylsulfoxide; ethers, such as 1,2-dimethoxyethane or diglyme; and carboxylic acids, for example acetic acid.

As also shown in scheme II, an intermediate acridinedione of formula VI can be prepared by reacting a compound of formula IX with an aldehyde of formula R⁴CHO, or acetal or hemiacetal thereof or a reactive derivative thereof. The reaction can be carried out at a temperature in the range of from 0 to 150°C, preferably at an elevated temperature, for example in the range of from 50 to 120 °C. Suitable solvents for the reaction include alcohols, for example, methanol or ethanol; dimethylsulfoxide; ethers, such as 1,2-dimethoxy ethane or diglyme; and carboxylic acids, for example acetic acid.

An intermediate of formula VII can be prepared by condensation of 1-morpholinocyclohexene and an aldehyde of formula R⁴CHO followed by hydrolysis as shown in Scheme III.

An intermediate of formula XII can be prepared by reacting an aldehyde of the formula R⁴CHO with the appropriate 1,3-cyclohexanedione. The reaction is conveniently effected at a temperature in the range of from 0° to 80°C, preferably 40° to 80°C. Suitable solvents for the reaction include alcohols, such as ethanol.

Pharmaceutically acceptable salts may be obtained using standard procedures well known in the art, for example by reacting a compound of formula I with a suitable acid or base affording a physiologically acceptable counterion.

When used to treat urinary incontinence, a compound of formula I is generally administered as an appropriate pharmaceutical composition which comprises a compound of formula I as defined hereinbefore together with a pharmaceutically acceptable diluent or carrier, the composition being adapted for the particular route of administration chosen. Such compositions are provided as a feature of the invention.

According to another aspect, therefore, the invention provides a pharmaceutical composition, which comprises a compound of formula I or a pharmaceutically acceptable salt thereof, as defined hereinabove, and a pharmaceutically acceptable diluent or carrier.

The compositions may be obtained employing conventional procedures and excipients and binders and may be in a variety of dosage forms. For example, they may be in the form of tablets, capsules, solutions or suspensions for oral administration; in the form of suppositories for rectal administration; in the form of sterile solutions or suspensions for administration by intravenous, intravesicular, subcutaneous or intramuscular injection or infusion; or in the form of a patch for transdermal administration.

The invention further provides a method for the treatment of urinary incontinence, comprising administering to a mammal in need of such treatment an effective amount of a compound of formula I as defined above or a pharmaceutically acceptable salt thereof.

Treatment using a compound according to the invention can be remedial or therapeutic as by administering a compound following the onset or development of urinary incontinence in a patient. Treatment can also be prophylactic or prospective by administering a compound in anticipation that urinary incontinence may develop, for example in a patient who has suffered from incontinence in the past.

According to a further aspect, the invention provides the use of a compound of formula I, as defined hereinabove, in the manufacture of a medicament for the treatment of urinary incontinence.

Because compounds according to the invention function to open cell potassium channels, they may also be useful as therapeutic agents in the treatment of other conditions or diseases in which the action of a therapeutic agent which opens potassium channels is desired or is known to provide amelioration. Such conditions or diseases include hypertension, asthma, peripheral vascular disease, right heart failure, congestive heart failure, angina, ischemic heart disease, cerebrovascular disease, glaucoma, renal cholic, disorders associated with kidney stones, irritable bowel syndrome, male pattern baldness, premature labor, and peptic ulcers.

The dose of compound of formula I which is administered will necessarily be varied according to principles well known in the art taking account of the route of administration, the severity of the incontinence condition, and the size and age of the patient. In general, a compound of formula I will be administered to a warm blooded animal (such as man) so that an effective dose is received, generally a daily dose of above 0.005, for example in the range of about 0.01 to about 10 mg/kg body weight. Preferably the compound is administered orally in this dose range.

It will be apparent to those skilled in the art that a compound of formula I can be co-administered with other therapeutic or prophylactic agents and/or medicaments that are not medically incompatible therewith. Compounds within the scope of the invention have not been found show any indication of untoward side-effects in laboratory test animals at several multiples of the minimum effective dose.

The actions of compounds of formula I as smooth muscle relaxants useful as therapeutic agents for the treatment of urinary incontinence through their action to open potassium channels and hyperpolarize the membrane potential in the bladder detrusor smooth muscle can be shown using suitably designed in vitro tests, such as the one described following. Compounds according to the invention have been found to be active at 30 µM (micromolar) or less in this test. Compounds exemplified herein have typically been found to exhibit an IC₅₀ on the order of 30 micromolar or less in the test. For example, the compound described in Example 9 exhibits an IC₅₀ of 8.11 micromolar in the test. "IC₅₀" is a well understood term and means the concentration of test compound which causes a 50% decrease in the in vitro contraction of the bladder tissue described in the following test.

Hale albino Hartley guinea pigs (450-500g) are sacrificed by carbon dioxide induced asphyxiation and quickly exsanguinated. The lower abdominal cavity is opened and the urinary bladder isolated. The bladder is cleaned of surrounding connective and adipose tissue, and the portion above the ureteral orifices is removed and washed in Krebs-Henseleit buffer solution of the following composition (in mH): NaCl 118.0, KCl 4.7, HgSO₄ 1.2, KH₂PO₄ 1.2, CaCl₂ 2.5, NaHCO₃ 25.0 and d-glucose 11.1. The solution is warmed to 37°C and gassed with 95% O₂ and 5% CO₂. With vigorous bubbling, the solution should have a pH value close to 7.4.

The dome of the washed bladder is cut off and discarded; the remaining bladder is placed on a gauze in a Petri dish containing the buffer solution. A mid-ventral longitudinal cut is made with scissors to open the bladder. The strips cut from the dome and the base edge are discarded. The remaining detrusor mid-section is cut into two horizontal strips with an approximate width of 2.0 mm. These two strips are further bisected at the mid-dorsal section, creating four strip of similar dimensions. Each strip thus contains both dorsal and ventral portions of the bladder.

The two ends of each individual strip are tied to a glass support rod and a force-displacement transducer (Grass model FR03), respectively, with 4-0 black braided silk suture.

The transducers are connected to a polygraph (Grass model 7E), which is calibrated at 5 mV/cm and the calibration checked for linearity with weights of 5 and 0.5 grams. The analog electrical output signals from the polygraph are digitized by a Modular Instrument Hicro 5000 signal processing system using Biowindow Data Acquisition Software, which is run under the Hicrosoft OS/2 operating system with an IBM-compatible PC.

The detrusor strips on the glass rod are secured in 20 ml tissue baths and allowed to equilibrate under a preload tension of 2 grams. During the following 45 to 60 min equilibration period, the tissue is washed with fresh buffer solution at 15 min interval, with the tension adjusted, if necessary, to 2 grams prior to washing. After the equilibration period, a priming dose of 15mM KCl (total concentration in the bath) is applied. The tissue is washed after 10 min and washed twice more at 15 min intervals with tension adjusted to 2 grams before each washing.

When the tissue relaxes to a steady state after the final washing, 15 mH KCl is again applied. Once the myogenic activity of the tissue reaches a steady state, the baseline data are acquired through the Biowindows Data Acquisition System by averaging 5 min of the myogenic data sampled at 32 Hz. Once the baseline is acquired, the experimental compounds are dosed in a cumulative manner in half log unit increments. The contact time for each dose is 10 min with the final 5 min being the period of time that the dose reponse data are required. If 30 µM of the test compound does not abolished the detrusor mechanical activity, then 30 µM cromakalim, a putative potassium channel opener, is dosed to establish a maximum response. The effect of the compound at each dose is expressed as % of the maximum inhibitory response, which is further normalized with respect to the corresponding effect of the compound vehicle control. The normalized response is then used to derive the IC₅₀ of the relaxant activity of the compound through the application of Harquardt's nonlinear iterative curve fitting technique to a standard dose-response function.

The ability of compounds according to the invention to open potassium channels in detrusor smooth muscle can be further demonstrated by a second in vitro test.

This second in vitro test is similar to the one described above with regard to tissue preparation and data acquisition.
However, the following exceptions are noted. In this second test, the contraction of the detrusor strips during priming and after the equilibration period is achieved with 80 mM instead of 15 mM KCl (total concentration in the bath). A sustained tension in the tissue is evident after this high KCl stimulation, because voltage-sensitive calcium channels have been rendered open to permit an influx of calcium into the cells and the development of tonic tension. This tension is totally abolished with 300 µM of papaverine, which is thereby used to establish the maximum response in this test.

Typical calcium channel blockers like nifedipine, nimodipine, isradipine, and verapamil are able to relax and reduce the myogenic activity of guinea pig detrusor strips in both tests by virtue of their blocking action on calcium channels. However, all of the aforementioned calcium channel blockers are more potent in the second test when 80 mM KCl is used, than in the first test where 15 mM KCl is used. In contrast, while the putative potassium channel opener cromakalim has a potent relaxant activity in the first test with an IC₅₀ in the range of 0.6 to 0.9 µM, it demonstrates insignificant relaxant activity in the second test at concentrations as high as 30 µM. Thus, the profile of a higher relaxant activity in the first test than in the second of compounds according to the invention indicates that the compounds are functioning as potassium channel openers.

The ability of the compounds according to the invention to act as potassium channel openers on bladder tissue may be further demonstrated by a standard test which measures the effect of test compounds on the rate of efflux of rubidium from the tissue.

It will be further appreciated by those skilled in the art that the efficacy of compounds according to the invention can be demonstrated by standard assays in vivo. The following is a description of such a standard test.

Hale Wistar rats weighing 450-550 grams are anesthetized with 20 mg/kg, i.p. Nembutal and 80 mg/kg, i.p. Ketamine. The trachea is cannulated to prevent airway obstruction. Body temperature is maintained by means of a heating pad. Arterial blood pressure and heart rate may be measured with a pressure transducer connected to a polyethylene tube (PE50) which has been inserted into the right carotid artery. The right jugular vein is cannulated for drug administration. The urinary bladder is exposed through a midline abdominal incision and emptied of urine by application of slight manual pressure. A catheter (PE 50) is inserted through the apex of the bladder dome around 3-4 mm into its lumen and tied with suture (4-0 silk) to prevent leakage. The bladder catheter is connected to a pressure transducer for the measurement of bladder pressure. The bladder is then placed back into the abdominal cavity and the incision is stitched closed except where the catheter exits the cavity. The bladder is allowed to equilibrate for approximately 15 minutes. After the equilibration period, the rats are infused with saline directly into the bladder at a rate of 0.05 ml/min for the entire time of the experiment. The bladder pressure is then monitored for the start of bladder contractions. When the contractions start, the animal is then allowed to stabilize its pattern of contractions around 30 to 45 minutes before drug administration.

The test compounds are given i.v. The efficacy of a test compound is measured by comparison to the known reference drug cromakalim (SmithKline-Beecham) which is administered i.v. over the dose range of 0.05 to 0.5 mg/kg.

The above in vivo assay enables an assessment of both the blood pressure and cystometric activity of test compounds. Blood pressure is measured immediately after drug injection and at 5, 15 and 30 minutes later. Micturition contractions are induced by a slow continuous infusion of saline directly into the bladder. The average change (in seconds from control) in the duration of the intercontraction interval (the time between contractions) over an approximate 20-min period is reported for each compound.

The following is a description of a test in vivo which is complimentary to the above described tests and which can be used to ascertain if a test compound is active and, additionally, if the test compound exhibits selectivity for the bladder without significant cardiovascular effects when dosed orally. The compound described in Example 1 is active and selective in this test when dosed orally at 3 mg/kg body weight.

Male Wistar rats (400 - 500 g) were anesthetized with 50 mg/kg Nembutal, i.p. For each rat, the abdominal region and the front and back of the neck were shaved and povidone-iodine was applied to the skin. For carotid catheterization, the left carotid artery was exposed via a small ventral cervical incision. The exposed area was flushed with a 2% lidocaine HCl solution to relax the vessel. The catheter, filled with 0.9% saline, was introduced approximately 2.4 cm into the artery so that its tip resided in the aortic arch. The distal end of the catheter was exteriorized at the nape of the neck, filled with heparin (1000 units/ml) and heat sealed. For bladder catheterization, the bladder was exposed through a midline abdominal incision. A trocar was passed through the abdominal muscle about 1 cm from the upper end of the incision and then tunneled subcutaneously to emerge through the skin at the back of the neck. A saline-filled catheter was passed through the trocar. A small opening in the bladder dome was created with an Accu-Temp cautery. The catheter was placed into the bladder and secured with a 4-0 silk ligature. The catheter was flushed with saline and patency was noted. The external end of the catheter was heat-sealed to prevent urine leakage. The abdominal muscles and the skin were sutured. Both catheters were threaded through a stainless steel anchor button (Instech), which was then sutured to the subcutaneous muscle at the point of exteriorization. The skin was sutured closed over the button. The animals were allowed to recover from anesthesia.

24 - 48 hours after surgery, each rat was placed in a metabolism cage and connected via the anchor button to an Instech spring tether and swivel system to protect the catheters from damage and to allow the animal free movement in the cage. The carotid catheter was connected to a Gould P23XL pressure transducer for blood pressure measurement. The bladder catheter was connected to a pump for saline infusion and to a pressure transducer by means of PE50 tubing and a 4-way stopcock. A toploading balance with a collection cup was placed under the cage for urine output measurement.

The rats were weighed, orally sham-dosed (dosing needle introduced, but no fluid expelled), and transvesical saline infusion (.18 ml/min) was begun and continued throughout the experiment. Variations in blood pressure, heart rate, intravesical pressure and urine output were recorded on either a Grass Polygraph or a Gould TA4000 recording system. The animals were allowed to equilibrate until the micturition pattern became consistent (approx. 45 - 90 min.). At this point, a basal level of each experimental parameter was recorded and the rats were administered by oral gavage the appropriate dose of compound (in a 75% PEG 400 - saline vehicle) in concentrations such that the volume was 1 ml/kg body weight. The effects of the compounds on experimental parameters were followed for five hours after administration.

Experimental results for both the interval. between contractions and also heart rates were expressed as the mean ± S.E.M. (Standard Error of Measures) % change from basal level, with each animal serving as its own control. MAP is expressed as mean ± S.E.H mm Hg change from basal level.

Compounds according to the invention are active in one or more of the above-described tests.

The invention will now be illustrated by the following examples in which, unless stated otherwise:
(i) temperatures are given in degrees Celsius (°C); operations were carried out at room or ambient temperature, that is, at a temperature in the range of 18-25 °C;
(ii) organic solutions were dried over anhydrous magnesium sulfate; evaporation of solvent was carried out using a rotary evaporator under reduced pressure (600-4000 pascals; 4.5-30 mm Hg) with a bath temperature of up to 60 °C;
(iii) chromatography means 'flash chromatography; reversed phase chromatography means flash chromatography over octadecylsilane (ODS)-coated support having a particle diameter of 32-74 µ, known as "PREP-40-ODS" (Art 731740-100 from Bodman Chemicals, Aston, PA, USA); Thin layer chromatography (TLC) was carried out on silica gel plates;
(iv) in general, the course of reactions was followed by TLC and reaction times are given for illustration only;
(v) melting points are uncorrected and (dec) indicates decomposition; the melting points given are those obtained for the materials prepared as described; polymorphism may result in isolation of materials with different melting points in some preparations;
(vi) final products had satisfactory proton nuclear magnetic resonance (NMR) spectra;
(vii) yields are given for illustration only and are not necessarily those which may be obtained by diligent process development; preparations were repeated if more material was required;
(viii) when given, NMR data is in the form of delta values for major diagnostic protons, given in parts per million (ppm) relative to tetramethylsilane (TMS) as an internal standard, determined at 300 MHz using perdeuterio dimethyl sulfoxide (DHSO-d₆) as solvent; conventional abbreviations for signal shape are used; coupling constants (J) are given in Hz; Ar designates an aromatic proton when such an assignment is made;
(ix) chemical symbols have their usual meanings; SI units and symbols are used;
(x) reduced pressures are given as absolute pressures in pascals (Pa); elevated pressures are given as gauge pressures in bars;
(xi) solvent ratios are given in volume:volume (v/v) terms; and
(xii) mass spectra (MS) were run with an electron energy of 70 electron volts in the electron impact (EI) mode using a direct exposure probe; where indicated ionization was effected by chemical ionization (CI) or fast atom bombardment (FAB); values for m/z are given; generally, only ions which indicate the parent mass are reported.

### Example 1. 2-Methyl-4-(3-nitrophenyl)-4,6,7,8-tetrahydro-5(1H)-quinolone.

2-Methyl-4-(3-nitrophenyl)-5-oxo-1,4,5,6,7,8-hexahydro-3-quinolinecarboxylic acid (4.66 g) was suspended in ethanol (200 mL) with concentrated sulfuric acid (0.5 mL). The mixture was heated at reflux for 5 h, during which time all of the solid went into solution. The solvent was evaporated, and the residue was partitioned between aqueous 2 N sodium hydroxide and ethyl acetate. The aqueous portion was extracted with ethyl acetate. The combined extracts were washed (water, brine), dried, and evaporated to yield a residue which was purified by chromatography, eluting with ethyl acetate, to afford the title compound as a pale yellow solid (1.6 g); mp 183-185 °C; MS: 284 (H); 250 MHz NMR: 1.76 (s,3, CH₃), 187 (m,2, CH₂), 2.16 (m,2, CH₂), 2.46 (m,2, CH₂), 4.58 (d,1, J=4.9, CH), 4.64 (d,1, J=4.9, CH), 7.53 (t,1, 7.6, Ar), 7.62 (d, 1, J=7.6, Ar), 7.96 (s,1, Ar), 7.98 (m,1, Ar), 8.80 (s,1, NH). Analysis for C₁₆H₁₆N₂O₃: Calculated: C, 67.59; H, 5.67; N, 9.85; Found: C, 67,44; H, 5.75; 9.56.

The starting 2-Methyl-4-(3-nitrophenyl)-5-oxo-1,4,5,6,7,8-hexahydro-3-quinolinecarboxylic acid was prepared as follows.
a. 2-Cyanoethyl 2-methyl-4-(3-nitrophenyl)-5-oxo-1,4,5,6,7,8-hexahydro-3-quinolinecarboxylate. 2-Cyanoethyl acetoacetate (4.27 g), 3-nitrobenzaldehyde (4.24 g), 1,3-cyclohexanedione (3.09 g) and ammonium acetate (4.26 g) were combined in ethanol (240 mL) and heated at reflux for 18 h. The solvent was evaporated and the residue was chromatographed, eluting with ethyl acetate, to afford the quinolinecarboxylate as a yellow solid (7.42 g); MS: 381 (M); 250 MHz NMR: 1.75-2.00 (m,2, CH₂), 2.22 (m,2, CH₂), 2.35 (s,3, CH₃), 2.81 (m,2, CH₂), 3.34 (m,2 CH₂), 4.12 (t,2, J=5.9, CH₂), 5.01 (s,1, CH), 7.51 (m,1, Ar), 7.84 (dd,1, J=7.7, 1.1, Ar), 7.97 (m,1, Ar), 7.99 (s,1, Ar), 9.43 (s,1, NH).
b. 2-Methyl-4-(3-nitrophenyl)-5-oxo-1,4,5,6,7,8-hexahydro-3-quinolinecarboxylic acid. To a suspension of 2-cyanoethyl 2-methyl-4-(3-nitrophenyl)-5-oxo-1,4,5,6,7,8-hexahydro-3-quinolinecarboxylate (0.47 g) in ethylene glycol dimethyl ether (2 mL) was added 1 N sodium hydroxide (4 mL). The mixture was stirred for 20 h, during which time all of the solid went into solution. The mixture was diluted with water, washed with ethyl acetate, and acidified by addition of concentrated hydrochloric acid. The resulting precipitate was collected by vacuum filtration and dried to give the acid as a yellow solid (0.31 g); mp 253-255 °C; MS: 328 (H); NMR: 1.63-2.00 (m,2, CH₂), 2.20 (m,2, CH₂), 2.31 (s,3, CH₃), 2.49 (m,2, CH₂), 5.01 (s,1, CH), 7.49-7.61 (m,2, Ar), 7.97 (m,2, Ar), 9.24 (s, 1, NH). Analysis for C₁₇H₁₆N₂O₅: Calculated: C, 62.19; H, 4.91; N, 8.53; Found: C, 62.16; H, 5.02; N, 8.33.

### Examples 2-4

Except as otherwise indicated, the following 4-aryl-2-methyl-4,6,7,8-tetrahydro-5(1H)-quinolones of formula I, in which R⁴ indicates the 4-aryl radical, were prepared from the corresponding 4-aryl-2-methyl-5-oxo-1,4,5,6,7,8-hexahydro-3-quinolinecarboxylic acids of formula III, using procedures similar to that described in Example 1, with exceptions as noted.

Example 2. R⁴=3-cyanophenyl; The product of Example 2.b. (1.10 g) was suspended in diethylene glycol (10 mL) and heated at 180°C for 25 min. The reaction mixture was purified by chromatography, eluting with ethyl-acetate, to provide the title compound as a pale yellow solid (0.52 g, 56%); mp 163-165°C; MS: 264 (H); NMR: 1.74 (s,3, CH₃), 1.86 (m,2, CH₂), 2.16 (m,2, CH₂), 2.44 (m,2, CH₂), 4.47 (d,1, J=4.8, CH), 4.60 (d,1, J=4.2, CH), 7.51 (m,4, Ar), 8.55 (s,1, NH). Analysis for C₁₇H₁₆N₂0·0.15 H₂O: Calculated: C, 76.47; H, 6.14; N, 10.49; Found: C, 76.36; H, 6.17; N, 10.45.

Example 3. R⁴=3-bromo-4-fluorophenyl; Chromatography afforded a solid which was triturated with diethyl ether, filtered and dried to provided the title compound as a yellow solid (1.52 g); mp 177-179°C; MS: 336 (H); 400 MHz NMR: 1.74 (s,3, CH₃), 1.75-1.90 (m,2, CH₂), 2.14 (m,2, CH₂), 2.43 (m,2, CH₂), 4.41 (d,1, J=4.7, CH), 4.58 (d,1, J=4.8, CH), 7.18 (m,2, Ar), 7.37 (dd,1, J=6.9, 2.0, Ar), 8,52 (s,1, NH). Analysis for C₁₆H₁₅BrFNO: Calculated: C, 57.16; H, 4.50; N, 4.17; Found: C, 57.11; H, 4.61; 4.07.

Example 4. R⁴=4-fluorophenyl; the reaction mixture was evaporated and the residue was taken up in aqueous sodium bicarbonate and extracted with ethyl acetate. The combined organic extracts were washed (water, brine) and evaporated. The residue was chromatographed, eluting with ethyl acetate, providing an oil which solidified on standing. The solid was triturated with diethyl ether containing a few drops of ethyl acetate. Filtration and drying provided the title compound as a pale yellow solid (0.17 g); mp 170-173 °C; MS: 257 (M); 250 MHz NMR: 1.72 (s,3, CH₃), 1.83 (m,2, CH₂), 2.13 (m,2, CH₂), 2..42 (m,2, CH₂), 4.38 (d,1, J=4.8, CH), 4.59 (d,1, J=4.9, CH), 7.04 (M,2, Ar), 7.14 (m,2, Ar), 8.45 (s,1, NH). Analysis for C₁₆H₁₆FNO: Calculated: C, 74.69; H, 6.27; N, 5.44; Found: C, 74.45, H, 6.32; N, 5.34.

The starting 4-aryl-2-methyl-5-oxo-1,4,5,6,7,8-hexahydro-3-quinolinecarboxylic acids for Examples 2 and 3 were prepared as follows.

Except as otherwise indicated, the following 4-aryl-2-methyl-5-oxo-1,4,5,6,7,8-hexahydro-3-quinolinecarboxylates of formula VIII in which Ra is 2-cyanoethyl and R⁴ indicates the 4-aryl radical, were prepared from the corresponding benzaldehyde derivatives of formula R⁴CHO using procedures similar to that described in Example 1.a, eluting with the indicated chromatography solvent.
2.a. R⁴=3-cyanophenyl; chromatography solvent: ethyl acetate; obtained as a pale yellow solid; MS: 361 (M); 250 MHz NMR: 1.70-2.00 (m,2, CH₂), 2.23 (m,2, CH₂), 2.35 (s,3, CH₃), 2.52 (m,2, CH₂), 2.82 (m,2, CH₂), 4.13 (t,2, J=5.7, CH₂), 4.94 (s,1, CH), 7.44 (m,1, Ar), 7.54 (m,3, Ar), 9.37 (s,1, NH).
3.a. R⁴=3-bromo-4-fluorophenyl; chromatography solvent: ethyl acetate; obtained as a yellow solid (77%); MS: 433 (M); NMR: 1.67-2.00 (m,2, CH₂), 2.20 (m,2, CH₂), 2.32 (s,3, CH₃), 2.50 (m,2, CH₂), 2.82 (m,2, CH₂), 4.13 (t,2, J=5.9, CH₂), 4.87 (s,1, CH), 7.18 (m,2, Ar), 7.38 (d,1, J=6.1, Ar), 9.33 (s,1, NH).
4.a. R⁴=4-fluorophenyl; chromatography solvent: ethyl acetate; obtained as a pale yellow solid (86%); MS, 354 (M) 250 MHz NMR: 1.63-2.00 (m,2, CH₂), 2.18 (m,2, CH₂), 2.29 (s,3, CH₃), 2.50 (m,2, CH₂), 2.81 (m,2, CH₂), 4.11 (t,2, J=5.4, CH₂), 4.87 (s,1, CH), 6.97 (t,2, J=8.8, Ar), 7.18 (m,2, Ar), 9.25 (s,1, NH).

Except as otherwise indicated, the following 4-aryl-2-methyl-5-oxo-1,4,5,6,7,8-hexahydro-3-quinolinecarboxylic acids of formula III, in which R⁴ indicates the 4-aryl radical, were prepared by hydrolysis of the corresponding 2-cyanoethyl esters of formula VIII, described above, using procedures similar to that described in Example 1.b.
2.b. R⁴=3-cyanoethyl; the reaction mixture was poured into ice water and as acidified with 1N hydrochloric acid. The resulting precipitate was collected by vacuum filtration and dried, providing the title compound as a pale yellow solid (95%) ; MS: 308 (M); NMR: 1.62-1.97 (m,2, CH₂), 2.19 (m,2, CH₂), 2.30 (s,3, CH₃), 2.49 (m,2, CH₂), 4.92 (s,1, CH), 7.47 (m,3, Ar), 7.56 (m,1, Ar), 9.17 (s,1, NH) 11.80 (broad s,1, CO₂H).
3.b. R⁴=3-bromo-4-fluorophenyl; the reaction mixture was acidified with 6 N hydrochloric acid. The resulting precipitate was collected by vacuum filtration and dried, providing the title compound as an off-white solid (69%); MS: 380 (H); 250 MHz NMR: 1.65-200 (m,2, CH₂), 2.21 (m,2, CH₂), 2.30 (s,3, CH₃), 2.51 (m,2, CH₂), 4.88 (s,1, CH), 7.20 (m,1, Ar), 7.36 (d,1, J=6.8, Ar), 9.17 (s,1, NH), 11.81 (broad s,1, CO₂H).
4.b. R⁴=4-fluorophenyl; obtained as a white solid (94%); mp 235-237 °C (dec); MS: 301 (H) 250 MHz NMR: 1.63-2.00 (m,2, CH₂), 2.19 (m,2, CH₂), 2.28 (s,3, CH₃), 2.47 (m,2, CH₂), 4.88 (s,1, CH), 7.00 (m,2, Ar), 7.15 (m,2, Ar), 9.09 (s,1, NH), 11.73 (broad s,1, CO₂H). Analysis for C₁₇H₁₆FNO₃: Calculated: C,67.76; H, 5.35; N, 4.65; Found: C, 67.63; H, 5.35: N, 4.61

### Example 5. 2-Methyl-4-phenyl-4,6,7,8-tetrahydro-5(1H)-quinolone.

trans-4-Phenyl-3-butene-2-one (1.63 g), 1,3-cyclohexanedione (1.30 g), and ammonium acetate (1.82 g) were combined in ethanol (100 mL) and heated at reflux for 5 h. The solvent was evaporated and the residue was chromatographed, eluting with ethyl acetate:hexane (2:1). A portion of the resulting solid was triturated with hot ethyl acetate, filtered, and dried to provide the tetrahydroquinolone as a pale yellow solid (0.53 g); mp 227-229°C; MS: 239 (H); 250 MHz NMR: 1.72 (s,3, CH₃), 1.73-1.95 (m,2, CH₂), 2.14 (m,2, CH₂), 2.43 (m,2, CH₂), 4.37 (d,1, J=4.8, CH), 4.60 (d,1 J=5.0, CH), 7.15 (m,5, Ar), 8.42 (s,1, NH). Analysis for C₁₆H₁₇NO: Calculated: C, 80.30; H, 7.16; N, 5.85; Found: C, 80.16; H, 7.18; N, 5.76.

### Example 6. 2-Methyl-4-(3,4-methylenedioxyphenyl)-4,6,7,8-tetrahydro-5(1H)-quinolone.

Using a procedure similar to that described in Example 5, but substituting 4-(3,4-Hethylenedioxyphenyl)-3-butene-2-one for trans-4-Phenyl-3-butene-2-one, the title compound was prepared. Chromatography, eluting with ethyl acetate, provided a yellow solid. A second chromatography, eluting with acetonitrile:dichloromethane (20:80)gave a solid, which was triturated with diethyl ether to give the tetrahydroquinolone (13%) as a pale yellow solid; mp 217-220°C; MS: 283 (H), 250 HHz NMR: 1.72 (s,3, CH₃), 1.84 (m,2, CH₂), 2.11 (m,2, CH₂), 2.42 (m,2, CH₂), 4.30 (d,1, J=4.7, CH), 4.57 (d,1, J=4.2, CH) 5.91 (s,2, CH₂), 6.58 (m,1, Ar), 6.65 (d,1, J=1.3, Ar), 6.73 (d,1, J=8.0, Ar), 8.42 (s,1, NH). Analysis for C₁₇H₁₇NO₃·0.1 H₂O: Calculated: C, 71.62; H, 6.08; N, 4.91; Found: C, 71.59; H, 6.16; N, 5.27.

### Example 7. 4-(4-Chlorophenyl)-2-methyl-4,6,7,8-tetrahydro-5(1H)-quinolone.

4-(4-Chlorophenyl)-3-butene-2-one (2.83 g), 1.3-cyclohexanedione (1.81 g), and ammonium acetate (2.60 g) were combined in ethanol (125 mL) and heated at reflux for 5.5 h. The solvent was evaporated, and the residue was taken up in water and extracted with ethyl acetate. The combined organic extracts were washed (water, brine), dried, and evaporated to yield a residue which was chromatographed, eluting with hexane:ethyl acetate (1:1) to yield the title compound as a pale yellow solid (1.46 g); mp 184-187 °C; MS: 273 (H); NMR: 1.73 (s,3, CH₃), 1.75-1.98 (m,2, CH₂), 2.15 (m,2, CH₂), 2.43 (t,2, J=6.0, CH₂), 4.39 (d,1, J=4.7, CH), 4.58 (d,1,J=4.8, CH), 7.14 (d,2, J=8.4, Ar), 7.26 (d,2, J=8.4, Ar), 8.48 (s,1, NH); Analysis for C₁₆H₁₆ClNO: Calculated: C, 70.20; H, 5.89; N, 5.12; Found: C, 70.21; H, 5.67; N, 5.02.

### Example 8. 2-Methyl-4-(3-trifluoromethylphenyl)-4,6,7,8-tetrahydro-5(1H)-quinolone.

A mixture of 4-(3-trifluoromethylphenyl)-3-butene-2-one (4.9 g), 1,3-cyclohexanedione (2.68 g), ammonium acetate (2.65 g) and 75 mL of ethanol were heated at reflux for eight hours and then cooled to room temperature. The solvent was evaporated and the residue was partitioned between water and ethyl acetate. The organic layer was dried, filtered, and evaporated to yield a yellow solid. Recrystallization from ethyl acetate provided the title compound (2.8 g) as an off-white solid; mp 184-185 °C; NMR: 1.74 (s,3, CH₃), 1.87-1.98 (m,2, CH₂), 2.13-2.19 (m,2, CH₂), 2.42-2.49 (m,2, CH₂), 4.50 (d,1, J=4.9, CH), 4.63 (d,1, J=4.9, CH), 7.45 (s,4, Ar), 8.53 (s,1, NH); (CI, CH₄) HS: m/z=308(M+1). Analysis for C₁₇H₁₆F₃NO: Calculated: C, 66.44; H, 5.25; N, 4.56; Found: C, 65.90; H, 5.33; N, 4.43.

### Example 9. 2-Methyl-4-(4-trifluoromethylphenyl)-4,6,7,8-tetrahydro-5(1H)-quinolone.

A mixture of 4-(4-trifluoromethyphenyl)-3-butene-2-one (4.9 g), 1,3-cyclohexanedione (2.68 g), ammonium acetate (2.65 g) and 75 mL of ethanol were heated at reflux for eight hours and then cooled to room temperature. The solvent was evaporated and the residue was partitioned between water and ethyl acetate. The organic layer was dried, filtered, and evaporated to obtain a yellow oil. Chromatography, with hexane:ethyl acetate (1:1) as the eluent, and recrystallization from toluene:hexane provided the title compound as a yellow solid (4.0 g); mp 116-118 °C; NMR: 1.73 (s,3, CH₃), 1.87-1.98 (m,2, CH₂), 2.14-2.16 (m,2, CH₂), 2.45-2.49 (m,2, CH₂), 4.49 (d,1, J=4.9, CH), 4.60 (d,1, J=4.9, CH), 7.35 (d,2, J=8.0, Ar), 7.57 (d,2, J=8.0, Ar), 8.54 (s,1, NH); (CI, CH₄) MS: m/z=308(M+1) ; Analysis for C₁₇H₁₆F₃NO: Calculated: C, 66.44; H, 5.25; N, 4.56; Found: C, 66.72; H, 5.34; N, 4.41.

### Example 10. 4-(3-Chlorophenyl)-2-methyl-4,6,7,8-tetrahydro-5(1H)-quinolone.

A mixture of 4-(4-chlorophenyl)-3-butene-2-one (5.0 g), 1,3-cyclohexanedione (3.24 g), ammonium acetate (3.20 g) and 90 mL of ethanol were heated at reflux overnight and then cooled to room temperature. The solvent was evaporated and the residue was partitioned between water and ethyl acetate. The organic layer was dried, filtered, and evaporated to obtain a yellow solid. Recrystallization from ethanol provided the title compound as a yellow solid (3.0 g); mp 201-203 °C; NMR: 1.73 (s,3, CH₃), 1.87-1.98 (m,2, CH₂), 2.13-2.17 (m,2, CH₂), 2.43-2.49 (m,2, CH₂), 4.40 (d,1, J=4.9, CH), 4.60 (d,1, J=4.9, CH), 7.09-7.25 (m,4, Ar), 8.50 (s,1, NH); (CI, CH₄) MS: m/z=274(M+1). Analysis for C₁₆H₁₆ClNO·0.15 H₂O: Calculated: C, 69.51; H, 5.94; N, 5.07; Found: C, 69.47; H, 6.03; N, 4.91.

### Example 11. 2-Hethyl-4-(4-methylphenyl)-4,6,7,8-tetrahydro-5(1H)-quinolone.

A mixture of 4-(4-methyphenyl)-3-butene-2-one (5.0 g), 1,3-cyclohexanedione (3.66 g), ammonium acetate ( 3.61 g) and 100 mL of ethanol were heated at reflux overnight and then cooled to room temperature. The solvent was evaporated and the residue was partitioned between water and ethyl acetate. The organic layer was dried, filtered, and evaporated to obtain an amber oil. Chromatography, with hexane:ethyl acetate (1:1) as the eluent, and trituration from ether provided the title compound as an off-white solid (2.0 g); mp 196-198 °C; NMR: 1.73 (s.3, CH₃), 1.87-1.98 (m,2, CH₂), 2.14-2.16 (m,2, CH₂), 2.22 (s,3, CH₃), 2.43-2.49 (m,2, CH₂), 4.34 (d,1, CH), 4.57 (d,1, CH), 7.01 (s,4, Ar), 8.40 (s,1, NH); (CI, CH₄) MS: m/z=254(M+1). Analysis for C₁₇H₁₉NO: Calculated: C, 80.60; H, 7.56; N, 5.53; Found: C, 80.24; H, 7.68; N, 5.36.

### Example 12. 9-(3-Nitrophenyl)-3,4,5,6,7,8,9,10-octahydro-1(2H)-acridinone.

A mixture of 2-(3-nitrophenylmel:hylene)cyclohexanone (4.69 g), 1,3-cyclohexanedione (2.28 g), ammonium acetate (2.35 g) and 90 mL of ethanol was stirred at reflux under nitrogen for 48 hours. The mixture was cooled in an ice bath and orange crystals were collected by filtration. The material was chromatographed with dichloromethane, ethyl ether:dichloromethane (2:98) and ethyl ether:dichloromethane (10:90) as the elutents. The solvent was evaporated and the residue recrystallized from toluene/hexane to yield the title compound as yellow crystals (0.50 g); mp 218-221 °C; NMR (CDCl₃): 1.57-1.59 (m,4, CH₂) 1.74-1.80 (m,2, CH₂) 1.88-2.01 (m,2, CH₂) 2.14 (broad s,2, CH₂) 2.28-2.36 (m,2, CH₂) 2.42-2.46 (m,2, CH₂) 4.50 (s,1, CH) 5.40 (s,1, NH) 7.38 (t,1, J=7.8, Ar) 7.71 (d,1, J=7.6, Ar) 7.98 (q,1, J=7.3, 1.4, Ar) 8.09 (d,1, J=1.9, Ar); MS (CI, CH₄): m/z=325(M+1). Analysis for C₁₉H₂₀N₂O₃·0.6 H₂O: Calculated: C, 68.08; H, 6.37; N, 8.36; Found: C, 68.27; H, 6.28; N, 8.01.
The starting 2-(3-nitrophenylmethylene)cyclohexanone was prepared as follows.

1-Morpholino-1-cyclohexene (13.28 g) was added dropwise to a stirred solution of 3-nitrobenzaldehyde (10.0 g) and 65 mL of dry toluene, under nitrogen. After stirring at room temperature for 5 days the mixture was treated with 75 mL of 5 N HCl, stirred an additional 15 minutes and the layers separated. The aqueous phase was extracted with three additional 75 mL portions of toluene, the toluene extracts were dried (MgSO₄), filtered and the solvent evaporated. The resulting yellow oil which turned greenish on standing overnight was chromatographed, with methylene chloride as elutent, to yield 2-(α-hydroxy-3-nitrobenzyl)cyclohexanone (6.35 g) as a yellow oil; MS (CI, CH₄): m/z=250(M+1). The yellow oil was dissolved in ethyl ether (50 mL) and treated with 50 mL of concentrated hydrochloric acid. After stirring overnight the layers were separated and the aqueous phase extracted with ethyl ether. The combined extracts were dried (MgSO₄), filtered and evaporated to yield crude 2-(3-nitrophenyl)methylenecyclohexanone as low melting orange crystals (4.69 g), which were used without further purification. NMR (CDCl₃): 1.79-1.89 (m,2, CH₂), 1.93-2.01 (m,2, CH₂), 2.56-2.60 (m,2, CH₂), 2.82-2.87 (m,2, CH₂), 7.47 (s,1, olefinic), 7.58 (t,1, J=7.9, Ar), 7.69 (d,1, J=7.7, Ar), 8.19 (d,1, J=8.9, Ar), 8.24 (s,1, Ar); MS (CI, CH₄) : m/z=232(M+1).

### Example 13. 9-(3-Cyanophenyl)-3,4,5,6,7,8,9,10-octahydro-1(2H)-acridineone.

A mixture of 9-(3-cyanophenyl)-3,4,6,7,9,10-hexahydro-1,8-(2H,5H)-acridinedione (3.18 g), sodium borohydride (2.50 g) and 50 mL of ethanol was stirred at 70 °C overnight. The mixture was treated with about 300 mL of water, stirred for several hours and the solid collected by suction filtration, slurried on the funnel with a little ethanol and sucked dry on the funnel. The solid (1.87 g) was combined with 0.49 g of material from a previous run and the combined material was chromatographed, with methanol:dichloromethane (2.5:97.5) as elutent. The solvent evaporated and the residue taken up in 50 mL of methanol and about 20 mL of methylene chloride. The solution was concentrated on a steam bath until crystals started to form, was treated with an equal volume of ethyl acetate and refrigerated overnight. The title compound was obtained as bright yellow crystals (1.16 g); mp 246-249 °C; NMR: 1.47 (broad s,2, CH₂), 1.65-1.87 (m,6, CH₂), 2.11 (m,4, CH₂), 2.39-2.51 (m,2, CH₂), 4.25 (s,1, CH), 7.41-7.57 (m,4, Ar), 8.45 (s,1, NH); (CI, CH₄) MS: m/z=305(M+1). Analysis for C₂₀H₂₀N₂O: Calculated: C, 78.92; H, 6.62; N, 9.20; Found: C, 78.64; H, 6.65; N, 9.08.

The intermediate 9-(3-cyanophenyl)-3,4,6,7,9,10-hexahydro-1,8-(2H,5H)-acridineone can be prepared as follows.

A stirred mixture of 3-cyanobenzaldehyde (1.48 g), 1,3-cyclohexanedione (2.53 g) and ammonium acetate (1.24 g) in ethanol (20 mL) was refluxed for 18 hours. The mixture was poured into water, and the yellow solid collected and dried under vacuum to yield the title acridinedione (3.22 g); mp 285-288 °C; NMR: 1.80-1.93 (m,4) 2.19-2.22 (m,4) 2.50-2.54 (m,4) 4.91 (s,1) 7.37-7.42 (m,1) 7.48-7.54 (m,3) 9.55 (s,1); MS: m/z=319 (M+1). Analysis for C₂₀H₁₈N₂O₂: Calculated: C, 75.44; H, 5.71; N, 8.80. Found: C,75.27; H, 5.66; N, 8.77.

### Example 14. 2-Isobutyl-4-phenyl-4,6,7,8-tetrahydro-5(1H)-quinolone.

5-Methyl-1-phenyl-1-hexen-3-one (5.05 g), 1,3-cyclohexanedione (3.31 g) and ammonium acetate (4.91 g) were combined in 200 mL of ethanol and allowed to reflux for 7 hours. The mixture was evaporated and the residue was purified by chromatography, with ethyl acetate as the eluent, to provide the title compound as a white solid (1.48-g); mp 182-184 °C; MS: 281 (M); NMR: 0.79 (d,3, J=5.5, CH₃), 0.85 (d,3, J=5.5, CH₃), 1.73-1.93 (m,5, CH₂, CH₂, CH), 2.13 (m,2, CH₂), 2.42 (m,2, CH₂), 4.38 (d,1, J=4.9, CH), 4.58 (d,1, J=5.0, CH), 7.03-7.23 (m,5, Ar), 8.30 (s,1, NH). Analysis for C₁₉H₂₃NO: Calculated: C, 81.80; H, 8.24; N, 4.98; Found: C, 80.91; H, 8.21; N, 5.00.

### Example 15. 2-Trifluoromethyl-4-(3-nitrophenyl)-4,6,7,8-tetrahydro-5(1H)quinolone.

A suspension of 3-carboxy-2-trifluoromethyl-2-hydroxy-4-(3-nitrophenyl)-4,6,7,8-tetrahydro-5(1H)-quinolone (3.17 g) in toluene (150 mL) was treated with p-toluenesulfonic acid monohydrate (0.32 g), the mixture refluxed vigorously under Dean-Stark conditions for 4 hours and then partitioned between water and ethyl acetate. The organic phase was washed (water and brine) and evaporated. The residue was purified by chromatography (hexane/ethyl acetate, 1:1 and methylene chloride/acetonitrile 95:5) to yield the title compound (0.83 g) as a pale yellow solid; mp 215-216°C; NMR: 1.77-2.00 (m,2, CH₂), 2.16-2.36 (m,2, CH₂), 2.50-2.71 (m,2, CH₂), 4.79 (d,1, J=4.1, CH), 5.67 (d,1, J=5.4, CH), 7.57-7.67 (m,2, Ar), 8.04 (m,2, Ar), 9.48 (s,1, NH); MS: m/z=338(M). Analysis for C₁₆H₁₃F₃N₂O₃: Calculated: C, 56.81; H, 3.87; N, 8.28; Found: C, 56.74; H, 4.02; N, 8.28.

The starting 3-carboxy-2-trifluoromethyl-2-hydroxy-4-(3-nitrophenyl)-4,6,7,8-tetrahydro-5(1H)-quinolone was obtained as follows:

A stirred mixture of ethyl 4,4,4-trifluoroacetoacetate (6.00 mL), 1,3-cylohexanedione (4.65 g), 3-nitrobenzaldehyde (6.31 g), and ammonium acetate (6.57 g) in ethanol (350 mL) was heated at reflux for 4.5 hours. After removal of solvent the orange residue was treated diethyl ether, and the resulting precipitate was collected by filtration. The solid was triturated with hot diethyl ether, collected, and purified by chromatography (ethyl acetate) to yield 3-carboethoxy-2-trifluoromethyl-2-hydroxy-4-(3-nitrophenyl) -4,6,7,8-tetrahydro-5(1H)-quinolone (8.47 g) as a white solid. NMR: 0.85 (t,3, J=7.1, CH₃), 1.86 (m,2, CH₂), 2.07 (m,2, CH₂), 2.27-2.44 (m,1, CH₂), 2.57-2.72 (m,1, CH₂), 2.75 (d,1, J=11.9, CH), 3.83 (m,2, CH₂), 4.07 (d,1, J=11.4, CH), 7.30 (s,1, OH), 7.50 (m,1, Ar), 7.57 (m,1, Ar), 7.87 (m,1, Ar), 8.01 (m,1, Ar), 8.16 (s,1, NH); MS: m/z=428(M).

A suspension of 3-carboethoxy-2-trifluoromethyl-2-hydroxy-4-(3-nitrophenyl)-2,3,4,6,7,8-hexahydro-5(1H)-quinolone (7.02 g) in ethanol (40ml) and water (40ml) was treated with lithium hydroxide monohydrate (1.44 g). The mixture was heated at 90°C for 30 minutes, diluted with water, acidified with 1N hydrochloric acid, and extracted with ethyl acetate. The combined organic extracts were washed (water and brine), dried, filtered and the solvent stripped to yield a brown oil which was further purified by chromatography (ethyl acetate and ethyl acetate/methanol, 4:1). 3-Carboxy-2-trifluoromethyl-2-hydroxy-4-(3-nitrophenyl)-4,6,7,8-tetrahydro-5(1H)-quinolone (2.07 g) was obtained as a white solid. NMR: 1.85 (m,2, CH₂), 2.10 (m,2, CH₂), 2.33-2.60 (m,3, CH₂, CH), 4.21 (d,1, J=8.7, CH), 7.47 (m,2, Ar), 7.81 (s,2, OH or NH, Ar), 7.96 (m, 1H, Ar); MS: m/z=400(H).

### Example 16. 2,7,7-Trimethyl-4-(3-nitrophenyl)-4,6,7,8-tetrahydro-5(1H)quinolone.

4-(3-Nitrophenyl)-3-buten-2-one (0.87 g), 5,5-dimethyl-1, 3-cyclohexanedione (0.66 g) and ammonium acetate (0.82 g) were combined in ethanol (30 mL) and heated at reflux for 6.5 hours. After removal of solvent and chromatography (hexane/ethyl acetate; 1:1) the title compound (1.02 g) was obtained as a yellow solid; mp 162-164°C; NMR: 0.94 (s,3, CH₃), 1.01 (s,3, CH₃), 1.76 (s,3, CH₃), 1.90-2.18 (m,2, CH₂), 2.25-2.43 (m,2, CH₂), 4.55 (d,1, J=4.7, CH), 4.64 (d,1, J=4.8, CH), 7.54 (m,1, Ar), 7.63 (m,1, Ar), 7.96 (s,1, Ar), 7.97 (m,1, Ar), 8.53 (s,1, NH); MS: m/z=312(M). Analysis for C₁₈H₂₀N₂O₃: Calculated: C, 69.21; H, 6.45; N, 8.97; Found: C, 69.07; H, 6.56; N, 8.85.

The starting 4-(3-Nitrophenyl)-3-buten-2-one was prepared as follows:

2N Sodium hydroxide (2.4 mL) was added dropwise at 5-10°C to a precooled (5-10°C) stirred solution of 3-nitrobenzaldehyde (10.04 g) in acetone (50 mL). The mixture was warmed to room temperature, stirred for 20 minutes, diluted with water, acidified with 2N hydrochloric acid and extracted with ethyl acetate. The combined organic extracts were washed (water and brine) and dried. Chromatography of the resulting orange oil (methylene chloride) gave the title compound (2.73 g) as a pale yellow solid. NMR: 2.37 (s,3, CH₃), 7.01 (d,1, J=16.0, CH), 7.73 (m,1, Ar), 7.78 (d,1, J=16.2, CH), 8.20 (d,1, J=7.9, Ar), 8.26 (m,1, Ar), 8.55 (s,1, Ar); MS: m/z=191(M).

### Example 17. 4-(3-Chlorophenyl)-2,7,7-trimethyl-4,6,7,8-tetrahydro-5(1H)quinolone.

A mixture of 4-(3-chlorophenyl)but-3-en-2-one (5.0 g), 5,5-dimethyl-1,3-cyclohexanedione (3.88 g), ammonium acetate (3.20 g) and ethanol (90mL) was heated at reflux for ten hours. The reaction mixture was worked up as described in Example 8 and recrystallization from ethyl acetate-hexane yielded the title compound (4.7 g) as a light yellow solid, mp 183-185°C; NMR: 0.94(s,3, CH₃), 1.00 (s,3, CH₃), 1.74 (s,3, CH₃), 1.95 (d,1, J=16, CH), 2.11 (d,1, J=16, CH), 2.25 (d,1, J=16.7, CH), 2.35 (d,1, J = 16.7, CH), 4.38 (d,1, J=4.6, CH), 4.59 (d,1, J=4.6, CH), 7.09-7.28 (m,4, Ar), 8.44 (s,1, NH); (CI, CH₄) MS: m/z=302(M+1). Analysis for C₁₈H₂₀ClNO: Calculated: C, 71.63; H, 6.68; N, 4.64; Found: C, 71.60; H, 6.69; N, 4.54.

### Example 18. 2-Ethyl-4-(3-nitrophenyl)-4,6,7,8-tetrahydro-5(1H)-quinolone.

A mixture of 1-(3-nitrophenyl)-1-penten-3-one (2.42 g), 1,3-cyclohexanedione (1.36 g), ammonium acetate (2.00 g) and ethanol (70 mL) was heated at reflux for 7.5 hours. Removal of solvent, chromatography (ethyl acetate/hexane, 2:1 and methylene chloride/acetonitrile, 9:1), trituration with hot diethyl ether and recrystallization from ethyl acetate provided the title compound (1.29 g) as-a yellow solid; mp 182-184°C; NMR: 1.03 (t,3, J=7.4, CH₃), 1.70-1.95 (m,2, CH₂), 2.07 (q,2, J=7.5, CH₂), 2.17 (m,2, CH₂), 2.48 (m,2, CH₂), 4.59 (d,1, J=4.8, CH), 4.66 (dd,1, J=4.8, 0.9, CH), 7.53 (m,1, Ar), 7.64 (m,1, Ar), 7.98 (m,2, Ar), 8.55 (s,1, NH). MS: m/z=298(M). Analysis for C₁₇H₁₈N₂O₃: Calculated: C, 68.44; H, 6.08; N, 9.39; Found: C, 68.43; H, 6.09; N, 9.39.
The starting 1-(3-Nitrophenyl)-1-penten-3-one was prepared as follows:

2-Butanone (3.90 mL) was added to a mixture of pyrrolidine (3.70 mL) and glacial acetic acid (2.50 mL) at 5°C. The cooling bath was removed, and a solution of 3-nitrobenzaldehyde (6.70 g) in toluene (30ml) and diethyl ether (10ml), was added dropwise to the mixture. Stirring was continued at room temperature for 48 hours. The reaction mixture was partitioned between water and ethyl acetate and acidified with 2N hydrochloric acid until the aqueous portion remained acidic. The organic portion was washed (water and brine) dried, the solvent stripped and the resulting brown solid chromatographed (hexane/ethyl acetate 2:1). Trituration with hexane/ethyl acetate gave the title compound (2.45 g) as a yellow solid; NMR: 1.04 (t,3, J=7.3, CH₃), 2.75 (q,2, J=7.3, CH₂), 7.10 (d,1, J=16.3, CH), 7.72 (m,1, Ar), 7.74 (d,1, J=16.3,CH), 8.20 (m,1, Ar), 8.25 (m,1, Ar), 8.56 (m,1, Ar); MS: m/z=205(M).

### Example 19. 2-Isopropyl-4-(3-nitrophenyl)-4,6,7,8-tetrahydro-5(1H)-quinolone.

4-Methyl-1-(3-nitrophenyl)-1-penten-3-one (2.90 g), 1,3-cyclohexanedione (1.50 g), and ammonium acetate (2.14 g) were combined in ethanol (100 mL) and heated at reflux for 7 hours. After removal of solvent, chromatography (hexane/ethyl acetate; 1:1) and trituration with diethyl ether the title compound (1.63 g) was obtained as a yellow solid; mp 195-196°C; NMR: 1.06 (d,3, J=6.9, CH₃), 1.07 (d,3, J=6.9, CH₃), 1.67-1.95 (m,2, CH₂), 2.07-2.22 (m,2, CH₂), 2.33 (m,1, CH), 2.49 (m,2, CH₂), 4.60 (d,1, J=5.0, CH), 4.67 (d,1, J=4.9, CH), 7.53 (m,1, Ar), 7.64 (m,1, Ar), 7.97 (m,2, Ar), 8.42 (s,1, NH); MS: m/z=312(M). Analysis for C₁₈H₂₀N₂O₃: Calculated: C, 69.21; H, 6.45; N, 8.97; Found: C, 69.03 ; H, 6.50; N, 8.60.

The starting 4-methyl-1-(3-nitrophenyl)-1-penten-3-one was obtained as follows:

3-Methyl-2-butanone (5.00 mL) was added to a mixture of pyrrolidine (3.40 mL) and glacial acetic acid (2.30 mL) at 5°C. The cooling bath was removed and a solution of 3-nitrobenzaldehyde (6.16 g) in toluene (30 mL) and diethyl ether (10 mL) was added dropwise to the mixture. After 48 hours at room temperature, the mixture was poured into 2N HCl and extracted with ethyl acetate. The organic phase was washed (water and brine), dried and the solvent removed. Purification by chromatography (hexane/ethyl acetate, 3:1) gave 1-hydroxy-4-methyl-1-(3-nitrophenyl)pentan-3-one (3.95 g) as a yellow solid. NMR: 0.96 (d,3, J=6.9, CH₃), 1.00 (d,3, J=6.9, CH₃), 2.62 (m,1, CH), 2.73-2.97 (m,2, CH₂), 5.14 (m,1, CH), 5.66 (d,1, J=4.7, OH), 7.62 (m,1,Ar),7.81 (d,1, J=7.7, Ar), 8.11 (m,1, Ar),8.23 (m,1, Ar); (CI, CH₄) MS: m/z=238(M+1).

A mixture of 1-hydroxy-4-methyl-1-(3-nitrophenyl)pentan-3-one (3.32 g), p-toluenesulfonic acid monohydrate (0.03 g) and toluene (50 mL) was refluxed for 1 hour under Dean-Stark conditions. The mixture was diluted with ethyl acetate, washed (sat. sodium bicarbonate, water and brine) and the solvent removed. Chromatography (hexane/ethyl acetate, 3:1) yielded 4-methyl-1-(3-nitrophenyl)-1-penten-3-one (2.98 g) as a yellow solid. NMR: 1.11 (d,6, J=6.8, CH₃), 3.02 (m,1, CH), 7.27 (d,1, J=16.2, CH), 7.73 (m,2, CH,Ar), 8.24 (m,2, Ar), 8.61 (m,1, Ar), MS: m/z=219(M).

### Example 20. 2-(t-Butyl)-4-(3-nitrophenyl)-4,6,7,8-tetrahydro-5(1H)-quinolone.

t-Butyl-3-nitrostyrylketone (1.81 g), 1.3-cyclohexanedione (0.82 g), and ammonium acetate (1.25 g) were combined in ethanol (50 mL) and heated at reflux for 7.5 hours. Following removal of solvent and chromatography (hexane/ethyl acetate; 1:1 and methylene chloride/acetonitrile 9:1) the title compound (0.54 g) was obtained as a yellow solid; mp 180-181.5°C; NMR: 1.12 (s,9, CH₃), 1.68-1.95 (m,2, CH₂), 2.06-2.27 (m,2, CH₂), 2.42-2.67 (m,2, CH₂), 4.61 (d,1, J=5.2, CH), 4.72 (dd,1, J=5.2, 1.8; CH), 7.53 (m,1, Ar), 7.64 (m,1, Ar), 7.97 (m,2, Ar), 8.10 (s,1, NH); MS: m/z=326(M). Analysis for C₁₉H₂₂N₂O₃: Calculated: C, 69.92; H, 6.79; N, 8.58; Found: C, 69.96; H, 6.80; N, 8.60.

### Example 21. 4-(3,4-Dichlorophenyl)-2-methyl-4,6,7,8-tetrahydro-5(1H)-quinolone.

A mixture of 1-(3,4-dichlorophenyl)but-1-en-3-one (5.0 g), 1,3-cyclohexanedione (2.71 g), ammonium acetate (2.70 g) and ethanol (75 mL) was heated at reflux for ten hours. The reaction was worked up as described in Example 8, and recrystallization from ethyl acetate-hexane yielded the title compound (2.6 g) as an off-white solid, mp 199-201°C; NMR: 1.74 (s,3, CH₃), 1.84-1.88 (m,2, CH₂), 2.14-2.19 (m,2, CH₂), 2.41-2.46 (m,2, CH₂), 4.42 (d,1, J=4.7, CH), 4.59 (d,1, J=4.7, CH), 7.12 (dd,1, J=8.3,2.0, Ar), 7.31 (d,1, J=2.0, Ar), 7.48 (d,1, J=8.3, Ar), 8.55 (s,1, NH); (CI, CH₄) MS: m/z=308(M+1). Analysis for C₁₆H₁₅Cl₂NO: Calculated: C, 62.35; H, 4.91; N, 4.54; Found: C, 62.03; H, 5.09; N, 4.44.

### Example 22. 4-(3-Methoxyphenyl)-2-methyl-4,6,7,8-tetrahydro-5(1H)-quinolone.

A solution of 1-(3-methoxyphenyl)but-1-en-3-one (2.07 g), 1,3-cyclohexanedione (1.37 g) and ammonium acetate (1.39 g) in ethanol (20 mL) was stirred at reflux for 10 hours. The reaction was worked up as described in Example 8 and purified by chromatography (10% v/v ethyl ether in methylene chloride) to yield the title compound (1.14 g) as an off-white solid. Recrystallization from ethanol/hexane gave analytically pure material, mp 164-167°C; NMR: 1.71 (s,3, CH₃), 1.82-1.88 (m,2, CH₂), 2.13-2.17 (m,2, CH₂), 2.40-2.44 (m,2, CH₂), 3.67 (s,3, CH₃), 4.35 (d,1, J=4.8, CH), 4.60 (d,1, J=4.6, CH), 6.64-6.66 (m,2, Ar), 6.71 (d,1, J=7.7, Ar), 7.12 (dd,1, J=7.7, 3.8, Ar), 8.41 (s,1, NH); (CI, CH₄)MS: m/z=270(M+1). Analysis for C₁₇H₁₉NO₂^{·}0.5H₂O: Calculated: C, 73.36; H, 7.24; N, 5.03. Found: C, 73.49; H, 6.98; N, 4.93.

### Example 23. 3-Acetyl-2-methyl-4-(3-nitrophenyl)-4,6,7,8-tetrahydro-5(1H)-quinolone.

4-Amino-3-penten-2-one (2.00 g), 1,3-cyclohexanedione (2.30 g), and 3-nitrobenzaldehyde (3.08 g) were combined in ethanol (180 mL) and heated at reflux for 5 hours. A yellow solid formed upon cooling to ambient temperature. The solid was purified by trituration with hot ethyl acetate/ethanol to yield the title compound (2.65 g) as a yellow solid; mp >250°C; NMR: 1.67-1.97 (m,2, CH₂), 2.13 (s,3, CH₃), 2.22 (m,2, CH₂), 2.35 (s,3, CH₃), 2.47 (m,2, CH₂), 5.11 (s,1, CH), 7.52 (m,1, Ar), 7.60 (d,1, J=7.7, Ar), 7.97 (m,2, Ar), 9.32 (s,1, NH); MS: m/z=326(M). Analysis for C₁₈H₁₈N₂O₄: Calculated: C, 66.25; H, 5.56; N, 8.58, Found: C, 65.99; H, 5.61; N, 8.40.

### Example 24. 2-(Isobutyl)-4-(3-nitrophenyl)-4,6,7,8-tetrahydro-5(1H)quinolone.

A solution of 5-methyl-1-(3-nitrophenyl)-1-hexen-3-one (2.52 g), 1,3-cyclohexanedione (1.22 g) and ammonium acetate (1.88 g) in ethanol (65 mL) was stirred at reflux for 7.5 hours. After removal of the solvent, the residue was chromatographed (ethyl acetate/hexane 2:1) to yield the title compound (1.51 g) as a yellow solid; mp 158-160°C; NMR: 0.81 (d,3, J=5.8, CH₃), 0.86 (d,3, J=5.8, CH₃), 1.77-1.97 (m,5, CH₂, CH), 2.10-2.23 (m,2, CH₂), 2.46 (m,2, CH₂), 4.59(d,1,J=4.8, CH), 4.64 (d,1, J=5.8, CH), 7.53 (m,1, Ar), 7.65 (m,1, Ar), 7.97 (m,2, Ar), 8.49 (s,1, NH); MS: m/z=326(H). Analysis for C₁₉H₂₂N₂O₃: Calculated: C, 69.92; H, 6.79; N, 8.58; Found: C, 70.03; H, 6.81; N, 8.54.

The starting 5-methyl-1-(3-nitrophenyl)-1-hexen-3-one was obtained as follows:

4-Methyl-2-pentanone (5.00 mL) was added to a mixture of pyrrolidine (3.30 mL) and glacial acetic acid (2.30 mL) at 5°C. The cooling bath was removed, and a solution of 3-nitrobenzaldehyde (6.12 g) in toluene (25 mL) and diethyl ether (10 mL) was added dropwise to the mixture. After 48 hours at room temperature, the reaction mixture was diluted with water, acidified with 2N hydrochloric acid and extracted with ethyl acetate. The combined organics were washed (water and brine), dried and the resulting oily residue purified by chromatography (hexane/ethyl acetate, 3:1) to yield the title compound (5.42 g) as a pale yellow solid. NMR: 0.92 (d,3, J=6.6, CH₃), 0.93 (d,3, J=6.6, CH₃), 2.14 (m,1, CH), 2.60 (d,2, J=7.0, CH), 7.09 (d,1, J=16.4, CH,), 7.72 (m,1, Ar), 7.75 (d,1, J=16.0, CH), 8.20 (d,1, J=7.9, Ar), 8.25 (m,1, Ar), 8.57 (s,1, Ar); MS: m/z=233(M).

### Example 25. 9-(3-Nitrophenyl)-3,4,5,6,7,8,9,10-octahydro-1(2H)-acridinone.

To a mixture of 9-(3-nitrophenyl)-3,4,6,7,9,10-hexahydro-1,8(2H,5H) -acridinedione (3.00 g) in ethanol (30 mL) was added sodium borohydride (2.21 g) in ethanol (20 mL). The mixture was heated at reflux for 16 hours, cooled, and additional sodium borohydride (1.11 g) was added. After an additional hour at reflux the reaction was filtered while hot and the filtrate diluted with water. Ethanol was removed and the aqueous portion extracted with ethyl acetate (2 x 150 mL). The combined organics were dried, concentrated to a brown oil and chromatographed (5-10% ethyl ether/methylene chloride) to yield the title acridinone as a yellow solid (1.12 g), mp 218-220°C; NMR: 1.46-1.52 (m,3, CH₂), 1.62-1.98 (m,5, CH₂), 2.05-2.19 (m,4, CH₂), 2.40-2.46 (m,2, CH₂), 4.35 (s,1, CH), 7.49-7.55 (m,1, Ar), 7.61-7.65 (m,1, Ar), 7.95-7.99 (m,2, Ar), 8.52 (s,1, NH); (CI, CH₄) MS: m/z=325(M+1). Analysis for C₁₉H₂₀N₂O₃: Calculated: C, 70.35; H, 6.21; N, 8.64; Found: C, 70.06; H, 6.25; ,; 8.34.

A preparation of the necessary acridinedione starting material is described in German patent application publication number DE 2003148.

### Example 26. 9-(3-Methoxyphenyl)-3,4,5,6,7,8,9,10-octahydro-1(2H)-acridinone.

To a mixture of 9-(3-methoxyphenyl)-3,4,6,7,9,10-hexahydro-1,8(2H,5H) -acridinedione (5.75 g) in ethanol (150 mL) was added sodium borohydride (4.04 g). The mixture was heated at reflux for 16 hours, cooled and additional sodium borohydride (2.69 g) was added. The mixture was heated at reflux for 8 hours, cooled and additional sodium borohydride (3.50 g) was added. The mixture was heated at reflux for 16 hours, poured into water and filtered to yield an off-white solid. Recrystallization from ethanol yielded the title acridinone as a white solid (5.36 g), mp 240-242°C; NMR: 1.46-1.52 (m,3, CH₂), 1.64-1.87 (m,5, CH₂), 2.08-2.13 (m,4, CH₂), 2.37-2.43 (m,2, CH₂), 3.68 (s,3, CH₃), 4.12 (s,1, CH), 6.63-6.74 (m,3, Ar), 7.10 (t,1, J=7.76, Ar), 8.34 (s,1, NH); (CI, CH₄) MS: m/z=310(M+1). Analysis for C₂₀H₂₃NO₂^{^{·}}0.50H₂O: Calculated: C, 75.44; H, 7.59; N, 4.40; Found: C, 75.20; H, 7.30; N, 4.37.

The necessary acridinedione starting material may be prepared according to the method described in S.M. Jain et al, Indian Journal of Chemistry, Volume 30B, November 1991, pages 1037-1040.

### Example 27. 9-(3-Trifluoromethoxyphenyl)-3,4,5,6,7,8,9,10-octahydro-1(2H)acridinone.

To a mixture of 9-(3-trifluoromethoxyphenyl)-3,4,6,7,9,10-hexahydro-1,8(2H,5H)-acridinedione (2.70 g), ethanol (25 mL) and dimethylformamide (15 mL) was added sodium borohydride (1.62 g). The mixture was heated at reflux for 16 hours, cooled and additional sodium borohydride (1.62 g) was added. The mixture was heated at reflux for 2 hours, cooled and additional sodium borohydride (2.00 g) was added. The mixture was heated at reflux for 2 hours, poured into water and filtered to yield an off-white solid. Purification by chromatography (5% ethyl acetate/methylene chloride) yielded the title acridinone as a white solid (1.41 g), mp 214-216°C. NMR: 1.46-1.52 (m,3, CH₂), 1.65-1.88 (m,5, CH₂), 2.08-2.14 (m,4, CH₂), 2.38-2.43 (m,2, CH₂), 4.23 (s,1, CH), 7.06 (m,2, Ar), 7.18 (d,1, J=7.73, Ar), 7.33 (m,1, Ar), 8.44 (s,1, NH); (CI, CH₄) MS: m/z=364(M+1). Analysis for C₂₀H₂₀NO₂F3^{^{·}}0.20H₂O: Calculated: C, 65.46; H, 5.60; N, 3.82; Found: C, 65.42; H, 5.53; N, 3.62.

The necessary acridinedione starting material may be prepared following the method described in Example 28 hereinbelow, but using 3-trifluoromethoxybenzaldehyde in place of 3-trifluoromethylbenzaldehyde. M.p. 273-275°C. Found for C₂₀H₁₈FNO₃: C,63.55; H,4.71; N,3.67.

### Example 28. 9-(3-Trifluoromethylphenyl)-3,4,5,6,7,8,9,10-octahydro-1(2H)-acridinone.

A mixture of 9-(3-trifluoromethylphenyl)-3,4,6,7,9,10-hexahydro-1,8(2H,5H)-acridinedione (2.0 g), sodium borohydride (2.1 g) and ethanol (50 mL) was heated at 70°C overnight and cooled to room temperature. The mixture was partitioned between water and ethyl acetate; the organic layer dried, filtered and concentrated. Chromatography (eluant: methylene chloride/methanol; 98/2) and recrystallization from ethanol/hexane provided the title compound (1.0 g) as a yellow solid, m.p. 250-251°C; NMR: 1.47-1.52 (m,3, CH₂), 1.66-1.90 (m,5, CH₂), 2.10-2.15 (m,4, CH₂), 2.40-2.45 (m,2, CH₂), 4.28 (s,1, CH), 7.45 (s,4, Ar), 8.44 (s,1, NH); (CI, CH₄) MS: m/z=348(M+1). Analysis for C₂₀H₂₀F₃NO: Calculated: C, 69.15; H, 5.80; N, 4.03; Found: C, 69.01; H, 5.83; N, 3.98.

The title compound was resolved into its two enantiomers by high pressure liquid chromatography, using a 250mm x 20mm column packed with a silica-based column packing with an ovomucoid stationary phase (Ultron ES-OVM) and eluting with 30% acetonitrile : 70% 0.013M KH₂PO₄ (adjusted to pH 5 with 1.0H KOH) at: a flow rate of 15.6ml/min. The enantiomers were detected by spectrophotometer at a wavelength of 250nm.

The necessary acridinedione starting material may be prepared as follows:-

A stirred mixture of 3-trifluoromethylbenzaldehyde (3.48 g), 1,3-cyclohexanedione (4.49 g), ammonium acetate (2.31 g) and ethanol (40 ml) was refluxed for 18 hours. The mixture was cooled and the yellow needles were collected, washed with water and dried in vacuo to give the title compound (6.38 g); mp >300 C; NMR: 1.7-2.0 (m,4), 2.19-2.25 (m,4), 2.50-2.56 (m,4), 4.98 (s,1), 7.41 (s,3), 7.48 (s,1), 9.48 (s,1); MS: m/z=362(M+1). Found for C₂₀H₁₈F₃NO₂: C, 66.44; H, 5.00; N, 3.80.

### Example 29. 9-(3-Fluorophenyl)-3,4,5,6,7,8,9,10-octahydro-1(2H)-acridinone.

A mixture of 9-(3-fluorophenyl)-3,4,6,7,9,10-hexahydro-1,8(2H,5H) -acridinedione (5.0 g), sodium borohydride (6.1 g), ethanol (140 mL) and pyridine (50 mL) was heated at 70°C overnight and cooled to room temperature. The solvent was removed and the resulting yellow solid was collected and washed well with water. Chromatography (ethyl acetate/hexane; 80/20) provided the title compound (2.1 g) as a yellow solid. Recrystallization from ethanol/hexane returned analytically pure light yellow solid, mp 249-251°C; NMR: 1.45-1.55 (m,3, CH₂), 1.66-1.90 (m,5, CH₂); 2.10-2.15 (m,4, CH₂), 2.39-2.45 (m,2, CH₂), 4.19 (s,1, CH), 6.87-6.92 (m,2, Ar), 7.00 (d,1, J=7.8, Ar), 7.20-7.27 (m,1, Ar), 8.41 (s,1, NH); (CI, CH₄) MS: m/z=298(M+1). Analysis for C₁₉H₂₀FNO: Calculated: C, 76.74; H, 6.78; N, 4.71; Found: C, 76.32; H, 6.71; N, 4.62.

The necessary acridinedione starting material may be prepared according to the method of Example 28 but using 3-fluorobenzaldehyde instead of 3-trifluoromethylbenzaldehyde. M.p.>350°C Found for C₁₉H₁₈NFO₂: C, 73.30, H,5.87; N,4.37.

### Example 30. 9-Phenyl-3,4,5,6,7,8,9,10-octahydro-1(2H)-acridinone.

A mixture of 9-phenyl-3,4,6,7,9,10-hexahydro-1,8(2H,5H)-acridinedione (5.0 g), sodium borohydride (6.5 g), ethanol (150 mL) and pyridine (50 mL) was heated at 70°C overnight and cooled to room temperature. The solvent was removed, the residue partitioned between water and ethyl acetate, the organic layer was dried and the solvent removed. Chromatography (methylene chloride/methanol; 98/2) provided the title compound (3.0g) as a white solid. Recrystallization from ethanol/hexane returned analytically pure title compound, mp 252-254°C, NMR: 1.41-1.51 (m,3, CH₂), 1.60-1.88 (m,5, CH₂), 2.03-2.13 (m,4, CH₂), 2.38-2.44 (m,2, CH₂), 4.14 (s,1, CH), 7.04-7.21 (m,5, Ar) 8.34 (s,1, NH); (CI, CH₄) MS: m/z=280(M+1). Analysis for C₁₉H₂₁NO^{^{·}}0.2H₂O: Calculated: C, 80.64; H, 7.62; N, 4.95; Found: C, 80.84; H, 7.62; N, 4.80.

The necessary acridinedione starting material may be prepared according to the method described in Example 28, but using benzaldehyde instead of 3-trifluoromethylbenzaldehyde.

### Example 31. 9-(3-Bromophenyl)-3,4,5,6,7,8,9,10-octahydro-1(2H)-acridinone.

To a stirred 70°C mixture of 9-(3-bromophenyl)-3,4,6,7,9,10-hexahydro -1,8(2H,5H)-acridinedione (5.0 g, 13.4), ethanol (120 mL) and pyridine (40 mL) was added sodium borohydride (7.5 g) in three portions over six hours. The solvent was removed; the resulting yellow solid washed well with water and air dried to provide the title compound (4.8 g). Recrystallization from ethanol/hexane returned analytically pure yellow solid, mp 258-260°C; NMR: 1.41-1.51 (m,3, CH₂), 1.60-1.88 (m,5, CH₂), 2.09-2.15 (m,4, CH₂), 2.39-2.45 (m,2, CH₂), 4.17 (s,1, CH), 7.16-7.21 (m,2, Ar), 7.26-7.29 (m,2, Ar), 8.44 (s,1, NH); (CI, CH₄) MS: m/z=360(M+1). Analysis for C₁₉H₂₀BrNO: Calculated: C, 63.69; H, 5.59; N, 3.91; Found: C, 63.73; H, 5.69; N, 3.94.

The necessary acridinedione starting material may be prepared as described in Example 28, but using 3-bromobenzaldehyde instead of 3-trifluoromethylbenzaldehyde. M.p. 336-339°C. Found for C₁₉H₁₈BrNO₂; C,61.12; H 4.94; N, 3.64.

### Example 32. 9-(3-Chlorophenyl)-3,4,5,6,7,8,9,10-octahydro-1(2H)-acridinone.

To a stirred 70°C mixture of 9-(3-chlorophenyl)-3,4,6,7,9,10-hexahydro-1,8(2H,5H)-acridinedione (5.0 g), ethanol (120 mL) and pyridine (40 mL) was added sodium borohydride (7.5 g) in two portions over six hours. The solvent was removed; the resulting yellow solid washed well with water and dried in vacuo to provide the title compound (5.0g) as a yellow solid. Recrystallization from ethanol returned analytically pure material, mp 249-250°C; NMR: 1.41-1.51 (m,3, CH₂), 1.60-1.88 (m,5, CH₂), 2.09-2.15 (m,4, CH₂), 2.39-2.45 (m,2, CH₂), 4.17 (s,1, CH), 7.09-7.15 (m,3, Ar), 7.21-7.26 (m,1, Ar), 8.43 (s,1, NH). (CI, CH₄) MS: m/z=314(M+1). Analysis for C₁₉H₂₀ClNO: Calculated: C, 72.72; H, 6.37; N, 4.46; Found: C, 72.55; H, 6.48; N, 4.49.

The necessary acridinedione starting material may be prepared as described in Example 28, but using 3-chlorobenzaldehyde instead of 3-trifluoromethylbenzaldehyde. M.p. >300°C. Found for C₁₉H₁₈ClNO₂: C,69.34; H,5.57; N,4.22.

### Example 33. 9-(4-Chlorophenyl)-3,4,5,6,7,8,9,10-octahydro-1(2H)-acridinone.

To a stirred 70°C mixture of 9-(4-chlorophenyl)-3,4,6,7,9,10-hexahydro-1,8(2H,5H)-acridinedione (6.0 g), ethanol (160 mL) and pyridine (50 mL) was added sodium borohydride (7.5 g, 198.4 mmole) in two portions over six hours. The solvent was removed; the resulting yellow solid washed well with water and dried in vacuo. Chromatography (methylene chloride/ethyl acetate; 85/15) and recrystallization from ethanol provided the title compound (1.0g) as a white solid, mp 253-254°C; NMR: 1.41-1.51 (m,3, CH₂), 1.60-1.88 (m,5, CH₂), 2.07-2.14 (m,4, CH₂), 2.38-2.41 (m,2, CH₂), 4.16 (s,1, CH), 7.16 (d,2, J=8.4, Ar), 7.25 (d,2, J=8.4, Ar), 8.39 (s,1, NH); (CI, CH₄) MS: m/z=314(M+1). Analysis for C₁₉H₂₀ClNO: Calculated: C, 72.72; H, 6.37; N, 4.46; Found: C, 72.35; H, 6.60; N, 4.29.

The necessary acridinedione starting material may be prepared as described in Example 28, but using 4-chlorobenzaldehyde instead of 3-trifluoromethylbenzaldehyde. H.p. >300°C, Found for C₁₉H₁₈N₂O₅.0.5H₂O; C,62.74; H,5.26; N,7.53.

### Example 34. 9-(3-Bromo-4-fluorophenyl)-3,4,5,6,7,8,9,10-octahydro-1(2H)-acridinone.

A mixture of 9-(3-bromo-4-fluorophenyl)-3,4,6,7,9,10-hexahydro-1,8(2H,5H)-acridinedione (1.8 g), sodium borohydride (1.74 g), ethanol (40 mL) and pyridine (14 mL) was heated at 70°C for three and one half hours. The solvent was removed, the residue partitioned between water and ethyl acetate, the organic layer dried and the solvent removed. Chromatography (methylene chloride/ethyl acetate; 7/3) gave the title compound (1.1 g), as a yellow solid, mp 254-257° C; NMR: 1.41-1.51 (m,3, CH₂), 1.60-1.88 (m,5, CH₂), 2.03-2.13 (m,4, CH₂), 2.38-2.44 (m,2, CH₂), 4.18 (s,1, CH), 7.13-7.24 (m,2, Ar), 7.38 (dd,1, J=6.9, 2.0, Ar), 8.45 (s,1, NH); (CI, CH₄) MS: m/z=378(M+1). Analysis for C₁₉H₁₉FBr: Calculated: C, 60.65; H, 5.09; N, 3.72; Found: C, 60.68; H, 5.09; N, 3.65.

The necessary acridinedione starting material may be prepared as described in Example 28, but using 3-bromo-4-fluorobenzaldehyde in place of 3-trifluoromethylbenzaldehyde. M.p. 308-310°C. Found for C₁₉H₁₇BrFNO₂: C,58.31; H,4.42; N,3.58.

### Example 35. 4-(3-Bromophenyl)-2-methyl-4,6,7,8-tetrahydro-5(1H)-quinolone.

A mixture of 3-bromobenzaldehyde (2.0 g), 1,3-cyclohexanedione (1.27 g) and ethanol (30 mL) was heated at reflux for three hours. Acetone (0.75 g), ammonium acetate (1.25 g) and a solution of pyrrolidine acetate (1.89 g) in 4 mL of ethanol was added and refluxing continued overnight. The mixture was cooled, filtered and the filtrate concentrated in vacuo. Chromatography (eluant: methylene chloride/methanol; 98/2) and trituration with ethyl ether provided the title compound (0.28 g) as a yellow solid, mp 187-191°C; NMR: 1.73 (s,3, CH₃), 1.80-1.90 (m,2, CH₂), 2.10-2.18 (m,2, CH₂), 2.40-2.45 (m,2, CH₂), 4.39 (d,1, J=4.7, CH), 4.59 (d,1, J = 4.7, CH), 7.12-7.28 (m,4, Ar), 8.50 (s,1, NH; (CI, CH₄) MS: m/z=320(M+1). Analysis for C₁₆H₁₆BrNO: Calculated: C, 60.39; H, 5.07; N, 4.40; Found: C, 60.19; H, 5.27; N, 4.10.

### Example 36. 4-(3-Trifluoromethoxyphenyl)-2-methyl-4,6,7,8-tetrahydro-5(1H)-quinolone.

A solution of 3-(trifluoromethoxy)benzaldehyde (10.00 g) and 1,3-cyclohexanedione (5.90 g) in ethanol (50 mL) was stirred at reflux for 3 hours. Ammonium acetate (6.09 g) and acetone (3.67 g) were added to the cooled mixture, followed by a solution of acetic acid (3.79 g) and pyrrolidine (4.49 g) in ethanol (15 mL). The mixture was stirred at reflux overnight, poured into water and extracted with ethyl acetate (2 x 200 mL). The combined organics were dried and concentrated to an oil which was purified by chromatography (0-40% v/v ethyl acetate in methylene chloride) to yield the title compound (2.36 g) as an off-white solid, mp 144-146°C; NMR: 1.74 (s,3, CH₃), 1.78-1.92 (m,2, CH₂), 2.14-2.19 (m,2, CH₂) 2.42-2.47 (m,2, CH₂) 4.46 (d,1, J=4.8, CH) 4.63 (d,1, J=4.9, CH) 7.01-7.05 (m,2, Ar) 7.17 (d,1, J=7.7, Ar) 7.36 (dd,1, J=7.7, 3.2, Ar), 8.52 (s,1, NH); (CI, CH₄) MS: m/z=324(M+1). Analysis for C₁₇H₁₆NO₂F₃: Calculated: C, 63.15; H, 4.99; N, 4.33; Found: C, 62.88; H, 5.05; N, 4.33.

### Example 37. 4-(3-Fluorophenyl)-2-methyl-4,6,7,8-tetrahydro-5(1H)-quinolone.

A mixture of 3-fluorobenzaldehyde (7.0 g), 1,3-cyclohexanedione (6.61 g) and ethanol (150 mL) was heated at 70°C overnight and then cooled to room temperature. Acetone (3.93 g), ammonium acetate (6.52 g) and a solution of pyrrolidine acetate (9.83 g) in ethanol (10 mL) was added and heated at 70° C for three hours and then cooled to room temperature. The solvent was removed and the residue was partitioned between water and ethyl acetate. The organic layer was dried, filtered, and concentrated in vacuo to obtain an amber oil. Chromatography (methylene chloride/ethyl acetate; 9.0/1.0) and recrystallization from ethanol provided the title compound (0.12 g) as a white solid, mp 216-218°C; NMR: 1.73 (s,3, CH₃), 1.78-1.90 (m,2, CH₂), 2.14-2.19 (m,2, CH₂), 2.41-2.46 (m,2, CH₂), 4.42 (d,1, J = 4.8, CH), 4.61 (d,1, J = 4.8, CH) 6.86-7.00 (m,3, Ar), 7.25 (m,1, Ar), 8.49 (s,1, NH); (CI, CH₄) MS: m/z=258(M+1). Analysis for C₁₆H₁₆FNO: Calculated: C, 74.69; H, 6.27; N, 5.44; Found: C, 74.39; H, 6.30; N, 5.35.

### Example 38. 3-Cyano-2-methyl-4-(3-nitrophenyl)-4,6,7,8-tetrahydro-5(1H) -quinolone.

3-Aminocrotonitrile (0.91 g), 1,3-cyclohexanedione (1.25 g), and 3-nitrobenzaldehyde (1.70 g) were combined in ethanol (100 mL) and heated at reflux for 7 hours. Removal of solvent and chromatography (ethyl acetate) gave the title compound (2.40 g) as a yellow solid; mp 214-216°C. NMR: 1.73-2.00 (m,2, CH₂), 2.08 (s,3, CH₃), 2.13-2.31 (m,2, CH₂), 2.41-2.63 (m,2, CH₂), 4.66 (s,1, CH), 7.61 (m,1, Ar), 7.68 (dd,1, J=7.7, 1.1 Ar), 7.98 (s,1, Ar), 8.07 (m,1, Ar), 9.66 (s,1, NH); MS: m/z=309(M). Analysis for C₁₇H₁₅N₃O₃: Calculated: C, 66.01; H, 4.89; N, 13.58; Found: C, 65.78; H, 4.99; N, 13.45.

### Example 39 2-Trifluoromethyl-4-(3-cyanophenyl)-4,6,7,8-tetrahydro-5(1H)-quinolone.

A suspension of 3-carboxy-2-trifluoromethyl-2-hydroxy-4-(3-cyanophenyl)- 4,6,7,8-tetrahydro-5(1H)-quinolone (2.25g) in toluene (100ml) was treated with p-toluene sulfonic acid monohydrate (0.33g). The mixture was vigorously refluxed under Dean-Stark conditions for 1.5 hours and then partitioned between water and ethyl acetate. The organic portion was washed (water and brine) and evaporated. The residue was purified by chromatography (hexane/ethyl acetate, 1:1) to yield the title compound (1.04g) as a white solid; m.p. 182-183°C; NMR: 1.80-2.00(m, 2, CH₂), 2.13-2.30(m, 2,CH₂), 2.50-2.67(m, 2,CH₂), 4.68(d, 1, CH, J = 5.2), 5.61(d, 1, CH, J = 5.3), 7.51(m, 2, Ar), 7.60(s, 1, Ar), 7.64(m, 1, Ar), 9.42(s, 1, NH); MS: m/z = 318(M); Analysis for C₁₇H₁₃F₃N₂O: Calculated: C, 64.15; H, 4.12; N, 8.80; Found: C, 64.15; H, 4.10; N, 8.63.

The starting 3-carboxy-2-trifluoromethyl-2-hydroxy-4-(3-cyanophenyl)-4,6,7,8-tetrahydro-5(1H)-quinolone was prepared as follows:-

A mixture of ethyl 4,4,4-trifluoroacetoacetate (5.0ml), 1,3-cyclohexanedione (3.87g), 3-cyanobenzaldehyde (4.61g), and ammonium acetate (5.63g) in ethanol (250ml) was refluxed for 5 hours. The resulting precipitate was collected by filtration (3.39g). The filtrate was concentrated and purified by chromatography (hexane/ethyl acetate) to provide additional product (5.77g). The 3-carboethoxy-2-trifluoromethyl-2-hydroxy-4-(3-cyanophenyl)-4,6,7,8-tetrahydro-5(1H)-quinolone product was obtained as a white solid; MS: m/z = 408(M); NMR: 0.86(t,3,CH₃, J = 7.0), 1.08-1.97(m,2,CH₂), 2.00-2.13(m,2,CH₂), 2.27-2.40(m,1,CH₂), 2.53-2.67(m,1,CH₂), 2.74(d,1,CH, J = 12.2), 3.77-3.80(m,2,CH₂), 3.98(d,1,CH, J = 11.7), 7.24(s,1,OH), 7.33-7.47(m,2,Ar), 7.50(s,1,Ar), 7.59(m,1,Ar) 8.10(s,1,NH).

A suspension of 3-carboethoxy-2-trifluoromethyl--2-hydroxy-4-(3-cyanophenyl)-4,6,7,8-tetrahydro-5(1H)-quinolone (3.39g) in ethanol (20ml) and water (20ml) was treated with lithium hydroxide monohydrate (0.75g). The mixture was heated at 90°C for 1.5 hours, diluted with water, acidified with 1N hydrochloric acid, and extracted with ethyl acetate. The combined organic extracts were washed (water and brine), dried, filtered, and evaporated to yield a foam which was purified by chromatography (ethyl acetate and ethyl acetate/methanol, 3:1) to afford 3-carboxy-2-trifluoromethyl-2-hydroxy-4-(3-cyanophenyl)-4,6,7,8-tetrahydro-5(1H)-quinolone (2.67g) as a pale yellow foam; MS: m/z = 381(m).

### Example 40 2-Methyl-4-(3-chloro-4-fluorophenyl)-4,6,7,8-tetrahydro-5(1H)-quinolone.

A mixture of 3-chloro-4-fluoro benzaldehyde (2.7g), 1,3-cyclohexanedione (1.99g) and 47mL of ethanol were heated at 50°C overnight and then cooled to room temperature. Acetone (1.18g), ammonium acetate (1.97g) and a premixed solution of pyrrolidine acetate (20.4 mmole) in 5mL of ethanol were added and heated at 70°C for three hours and then cooled to room temperature. The solvent was evaporated in vacuo and the residue was partitioned between water and ethyl acetate. The organic layer was dried over magnesium sulfate, filtered, and concentrated in vacuo. Chromatography (eluant: ether/hexane; 9.0/1.0) over silica gel provided the title compound 0.140g as a yellow solid, m.p. 164-167°C. 1H-NMR(300MHz, d6-DHSO): 1.74(s, 3H,CH₃), 1.81-1.88(m, 2H, CH₂) 2.15-2.19(m,2H, CH₂), 2.41-2.48(m,2H,CH₂), 4.41 (d,1H, J = 4.8Hz, CH), 4.60(d, 1H, J = 4.8(Hz,CH), 7.10-7.28(m, 3H, aromatic), 8.54(s, 1H, NH); MS (C1, CH4): 292 (M+1): Analysis for C₁₆H₁₅ClFNO: Calculated: C; 65.87; H; 5.18; N; 4.80: Found: C; 65.67; H; 5.50; N; 4.48.

### Example 41

### 9-(3-Trifluoromethyl-4-cyanophenyl)-3,4,5,6,7,8,9,10-octahydro-1-(2H)-acridinone.

A mixture of 9-(3-trifluoro-4-cyanomethylphenyl)-3,4,6,7,9,10-hexahydro-1,8-(2H,5H)-acridinedione (0.75g) and sodium borohydride (0.73g) and 17mL of ethanol and 6mL of pyridine were heated at 70°C for 3 hours and then coooled at room temperature. The solvent was removed in vacuo and the residue was partitioned between water and ethyl acetate. The organic layer was dried over magnesium sulfate, filtered and concentrated in vacuo. Chromatography (eluant: methylene chloride/ethyl acetate; 9.0/1.0 and trituration from ether/hexane provided the title compound (11g) as a yellow solid; m.p. 210-212°C; 1H-NMR (300 MHz, d6-DMSO): 1.47-1.89 (m, 8H, CH2), 2.05-2.16 (m, 4H, CH2), 2.40-2.45 (m, 2H, CH2), 4.41 (s, 1H, CH), 7.63 (d, 1H, J = 8.0Hz, aromatic), 7.74 (s, 1H, aromatic) 8.04 (d, 1H, J=8.0Hz, aromatic) 8.59 (s, 1H, NH); MS (CI, CH₄) : 373 (M+1); Analysis for C₂₁H₁₉F₃N₂O, Calculated : C; 67.73; H; 5.14; N; 7.52; Found : C, 67.76; H; 5.31; N; 7.46.

The necessary acridineodione starting material may be prepared as described in Example 28, but using 4-cyano-3-trifluoromethylbenzaldehyde; m.p. 289-291°C, Found C₂₁H₁₇F₃N₂O₂ : C, 65.08; H, 4.40;, 7.22.

### Example 42 9-(3-Chloro-4-fluorophenyl)-3,4,5,6,7,8,9,10-octahydro-1-(2H)-acridinone.

A mixture of 9-(3-chloro-4-fluorophenyl)-3,4,6,7,9,10-hexahydro-1,8-(2H,5H)-acridinedione (1.5g) and sodium borohydride (1.64g) and 38mL of ethanol and 13mL of pyridine were heated at 70°C for 3.5 hours and then cooled to room temperature. The solvent was removed in vacuo and the residue was partitioned between water and ethyl acetate. The organic layer was dried over magnesium sulfate, filtered, and concentrated in vacuo. Chromatography (eluent : methylene chloride/ethyl acetate; 8.0/2.0) over silica gel provided the title compound (0.75g) as a yellow solid, m.p. 236-239°C. 1H-NMR (300MHz, d6-DHSO): 1.41-1.51(m,3H, CH2) 1.60-1.88(m,5H,CH₂), 2.09-2.15(m,4H CH2) 2.38-2.44(m,2H,CH2), 4.19(s,1H,CH), 7.10-7.16(m,1H, aromatic), 7.21-7.27(m,2H, aromatic) 8.45 (s,1H,NH), MS(CI, CH₄): 332(M+1); Analysis for C₁₉H₁₉FClNO. 0.1H₂O. Calculated: C; 68.40; H; 5.80; N; 4.20. Found: C; 68.30; H; 5.84; N; 4.05.

The necessary acridineolione starting material may be prepared as described in Example 28, but using 3-chloro-4-fluorobenzaldehyde; m.p. 325-328°C; Found for C₁₉H₁₇ClFNO₂: C, 65.78; H, 4.88; N, 3.98.

Example 43. The following illustrate representative pharmaceutical dosage forms containing a compound of formula I, for example as illustrated in any of the previous Examples, (hereafter referred to as "compound X"), for therapeutic or prophylactic use in humans:

| (a) Tablet | |
|---|---|
| | mg/tablet |
| Compound X | 50.0 |
| Mannitol, USP | 223.75 |
| Croscarmellose sodium | 6.0 |
| Maize starch | 15.0 |
| Hydroxypropylmethylcellulose (HPHC), USP | 2.25 |
| Magnesium stearate | 3.0 |

| (b) Capsule | |
|---|---|
| Compound X | 10.0 |
| Hannitol, USP | 488.5 |
| Croscarmellose sodium | 15.0 |
| Magnesium stearate | 1.5 |

The above formulations may be obtained by conventional procedures well known in the pharmaceutical art. The tablets may be enteric coated by conventional means, for example to provide a coating of cellulose acetate phthalate.

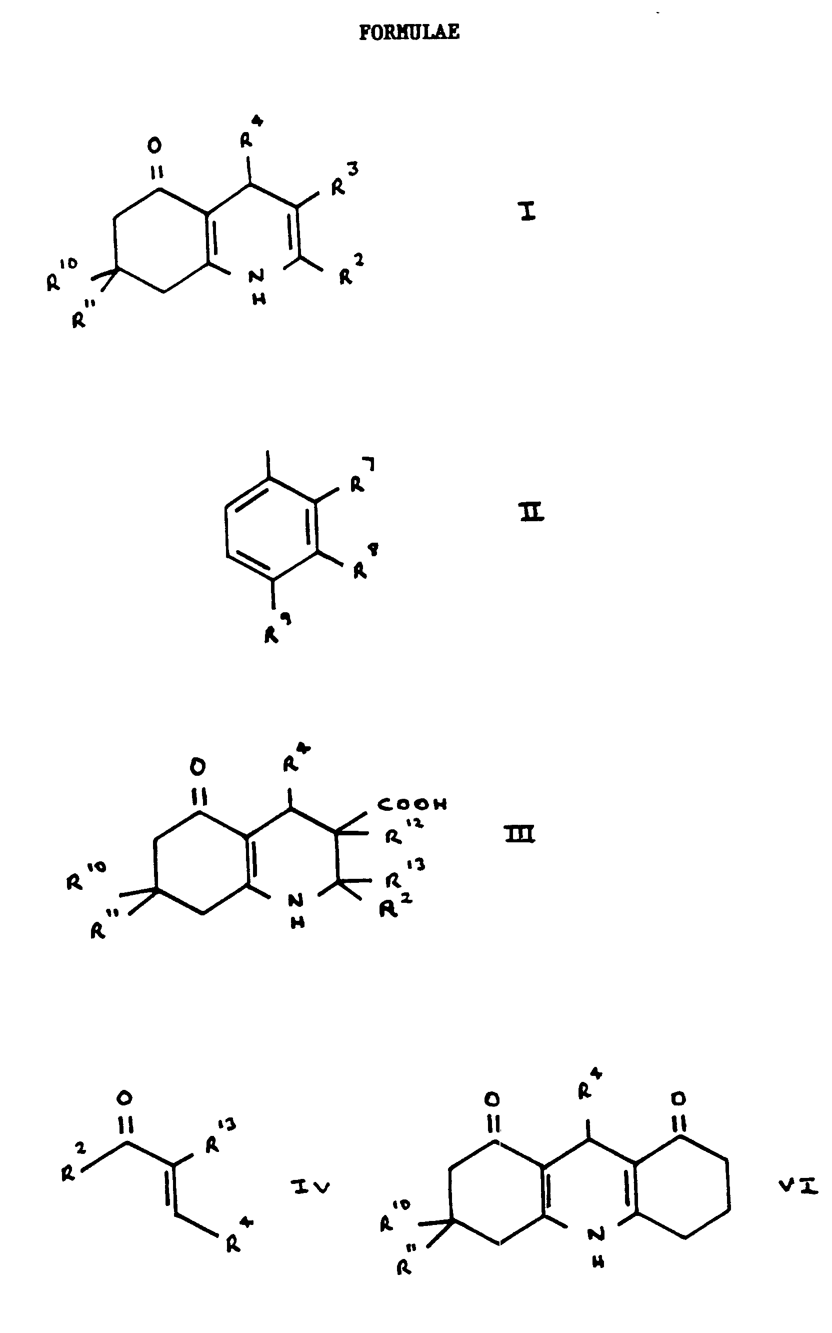

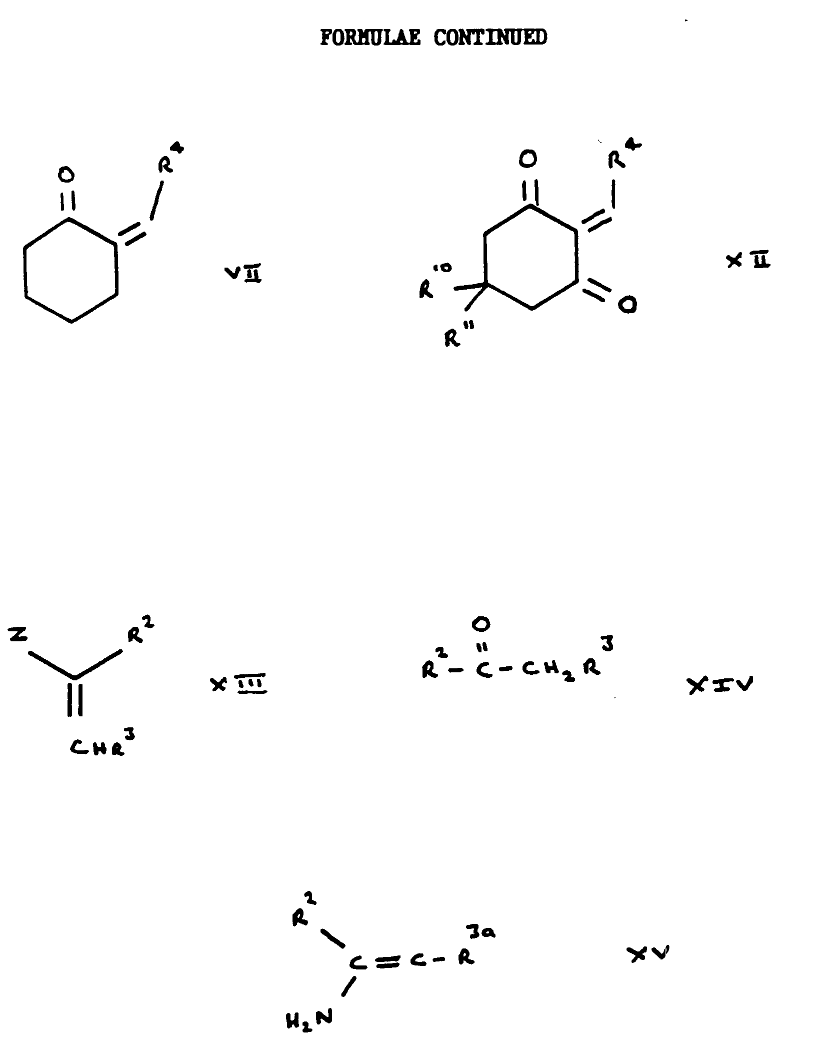

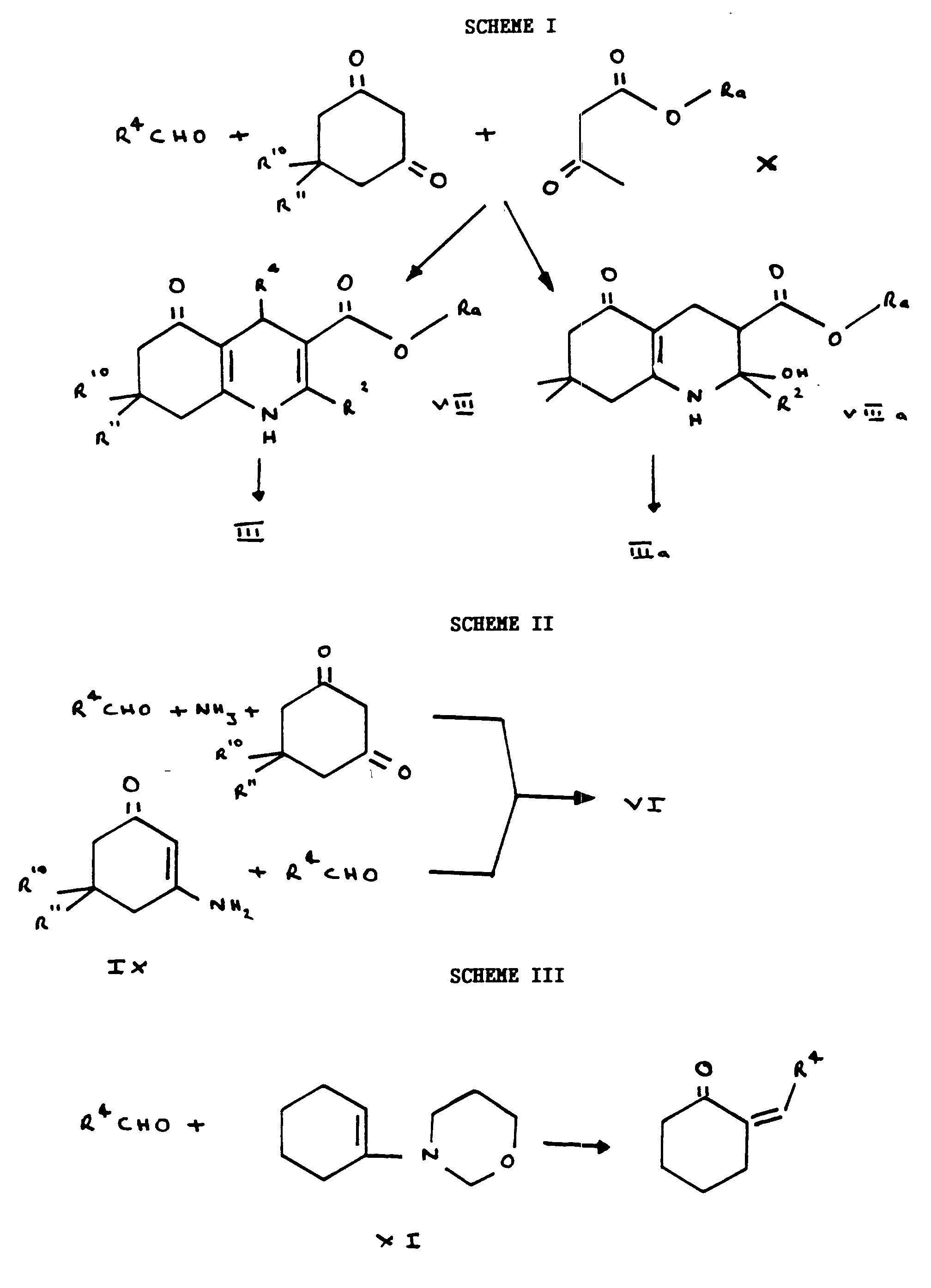

## Claims

1. A compound of formula I: 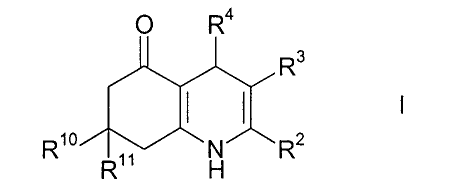 or a pharmaceutically acceptable salt thereof, wherein: either
R² is hydrogen, (1-6C)alkyl or (1-4C) fluoroalkyl; and
R³ is hydrogen, cyano, (1-6C)alkyl, (1-6C) fluoroalkyl or ethanoyl; or
R² and R³ when taken together form a 1,4-butandiyl;
R⁴ is 2- or 3-thienyl or furyl substituted at the 4- and/or 5-position(s) by a radical or radicals independently selected from a group (a) consisting of nitro, cyano, halo, (1-4C)alkyl, (1-4C)alkylsulphonyl and 2-thienyl provided that a 3-thienyl or furyl group may only be substituted at the 5-position; or
R⁴ is a 2-pyridyl which is substituted at the 5 position and/or either at the 4 position or the 6 position by a member of the above group (a); or
R⁴ is a 3-pyridyl which is substituted at the 6 position by a member of the above group (a); or
R⁴ is a 4-pyridyl which is substituted at the 2 position by a member of the above group (a); or
R⁴ is a group of formula II : 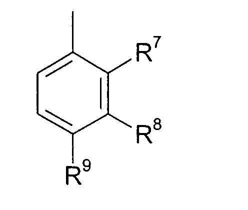 wherein :
R⁷ is hydrogen; and
R⁸ and R⁹ are independently selected from hydrogen, hydroxy (1-4C)alkoxy, nitro, cyano, (1-4C)fluoroalkyl, (1-4C)fluoroalkoxy, halo, (1-4C)alkyl, (1-4C)alkanoyl, phenyl and (1-4C)alkylsulphonyl; or
R⁸ and R⁹ taken together are (1-3C)alkylenedioxy; and
R¹⁰ and R¹¹ are each independently hydrogen or (1-4C) alkyl, but excluding 3-cyano-4-phenyl-2,7,7-trimethyl-4,6,7,8-tetrahydro--5(1H)-quinolone and 3-ethanoyl-4-phenyl-2,7,7-trimethyl-4,6,7,8-tetrahydro-5(1H)-quinolone.

2. A compound as claimed in claim 1, in which either
R² is methyl, ethyl, propyl, isopropyl, butyl, isobutyl, sec-butyl, tert-butyl or trifluoromethyl; and
R³ is hydrogen, cyano, ethanoyl; or
R² and R³ when taken together form a 1,4-butandiyl.

3. A compound as claimed in claim 2, in which R³ is hydrogen; or R² and R³ when together form a 1,4-butandiyl.

4. A compound as claimed in claim 3, in which R² is trifluoromethyl.

5. A compound as claimed in any one of claims 1 to 4, in which R⁴ is bromo-2-thienyl, 5-bromo-2-thienyl, 5-methylsulphonyl-2-thienyl, 5-methyl-2-thienyl, 5-(2-thienyl)-2-thienyl, 4-nitro-2-thienyl, 5-nitro-2-thienyl, 4-cyano-2-thienyl, and 5-nitro-3-thienyl or is a group of formula II in which R⁸ is selected from hydrogen, hydroxy, methoxy, nitro, cyano, trifluoromethyl, trifluoromethoxy, methyl, ethyl, isopropyl, halo and ethanoyl, and R⁹ is selected from hydrogen, hydroxy, methoxy, nitro, cyano, trifluoromethyl, trifluoromethoxy, methyl, ethyl, isopropyl and halo.

6. A compound as claimed in any one of claims 1 to 4, in which R⁴ is phenyl, 3-methoxyphenyl, 3-nitrophenyl, 3-cyanophenyl, 3-trifluoromethylphenyl, 3-trifluoromethyl-4-cyanophenyl, 4-trifluoromethylphenyl, 3-trifluoromethoxyphenyl, 3-fluorophenyl, 3-chlorophenyl, 3-chloro-4-fluorophenyl, 3-bromophenyl, 4-fluorophenyl, 4-chlorophenyl, 3-bromo-4-fluorophenyl, 3,4-dichlorophenyl, 4-methylphenyl, 3,4-methylenedioxyphenyl and 4-nitro-2-thienyl.

7. A compound as claimed in claim 6, in which R⁴ is 3-nitrophenyl or 3-cyanophenyl.

8. A compound as claimed in any one of claims 1 to 7, in which R¹⁰ and R¹¹ are both hydrogen or both methyl.

9. A compound as claimed in claim 1, in which either
R² is methyl, ethyl, propyl, isopropyl, butyl, isobutyl, sec-butyl, tert-butyl or trifluoromethyl; and
R³ is hydrogen; or
R² and R³ when taken together form a 1,4-butandiyl;
R⁴ is 3-nitrophenyl or 3-cyanophenyl; and
R¹⁰ and R¹¹ are both hydrogen.

10. A compound as claimed in claim 1, which is selected from 2-trifluoromethyl-4-(3-nitrophenyl)-4,6,7,8-tetrahydro-5(1H)-quinolone and 2-trifluoromethyl-4-(3-cyanophenyl)-4,6,7,8-tetrahydro-5(1H)-quinolone.

11. A process for the preparation of a compound of formula I or a pharmaceutically acceptable salt thereof as defined in claim 1, which comprises
(a) for a compound of formula I wherein R³ is hydrogen, by decarboxylation of a corresponding carboxylic acid of formula III 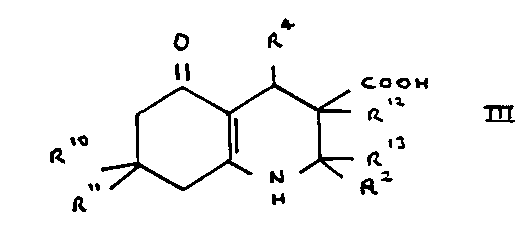 in which R¹² and R¹³ together represent a bond, or decarboxylation and dehydration of a carboxylic acid of formula III in which R¹² is hydrogen and R13 is a hydroxy group.
(b) by reacting an unsaturated ketone of formula IV 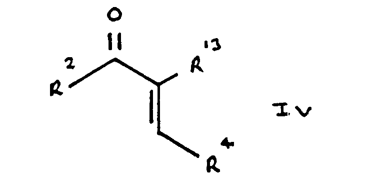 with the appropriate 1,3-cyclohexanedione and ammonia or an ammonium salt.
(c) for a compound of formula I wherein R² and R³ when taken together form a 1,4-butandiyl, by reacting an acridinedione of formula VI 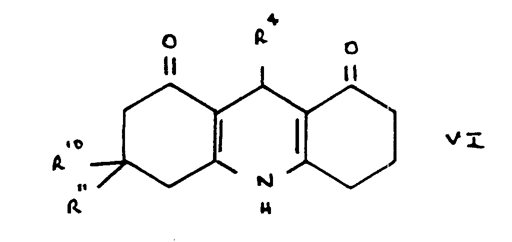 with a reducing agent.
(d) for a compound of formula I wherein R² and R³ when taken together form a 1,4-butandiyl, by reacting a ketone of formula VII 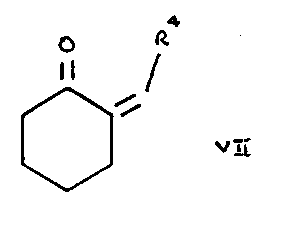 with the appropriate 1,3-cyclohexanedione and ammonia or an ammonium salt.
(e) by reacting an enedione of formula XII 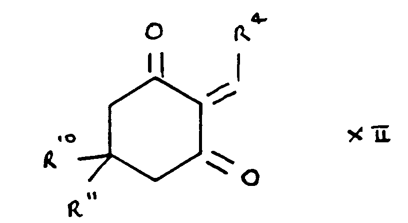 with enamine of formula XIII 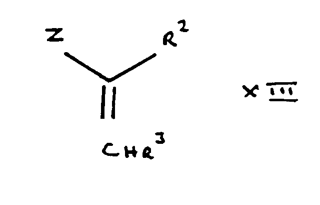 in which Z is an imino group in the presence of ammonia or an ammonium salt.
(f) for a compound of formula I where R³ is ethanoyl or cyano reacting a compound of formula XV 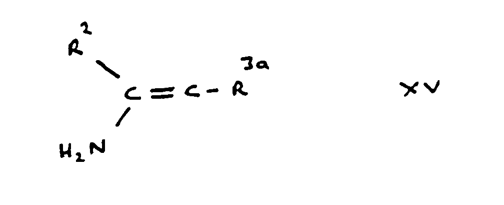 in which R^{3a} is ethanoyl or cyano with the appropriate 1,3-cyclohexanedione and the appropriate aldehyde of formula R⁴CHO,
wherein, if an optically active compound of formula I is desired, the compound of formula I is prepared using an optically active starting material or a racemic form of the compound of formula I is resolved, and
if a pharmaceutically acceptable salt is desired, a compound of formula I is reacted with a suitable acid or base affording a physiologically acceptable counter-ion; and
wherein R², R³, R⁴, R¹¹ and R¹² have the meanings given in claim 1.

12. A pharmaceutical composition, which comprises a compound of formula I or a pharmaceutically acceptable salt thereof, as defined in claim 1, and a pharmaceutically acceptable diluent or carrier.

13. The use of a compound of formula I or a pharmaceutically acceptable salt thereof as defined in claim 1 in the manufacture of a medicament for the treatment of urinary incontinence.

14. A compound of general formula III 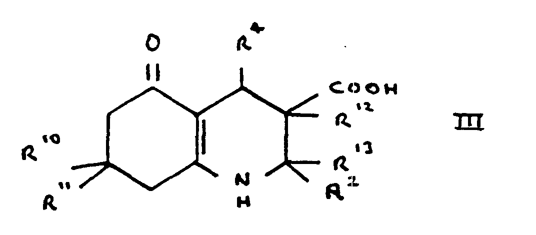 in which either R¹² and R¹³ together represent a bond or R¹² is hydrogen and R¹³ is a hydroxy group, and R², R⁴, R¹⁰ and R¹¹ are as defined in claim 1.

## Patentansprüche

1. Verbindung der Formel I: 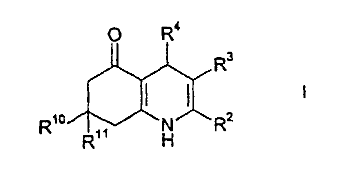 oder ein pharmazeutisch unbedenkliches Salz davon, wobei entweder
R² für Wasserstoff, C₁₋₆-Alkyl oder C₁₋₄-Fluoralkyl und
R³ für Wasserstoff, Cyano, C₁₋₆-Alkyl, oder C₁₋₆-Fluoralkyl oder Ethanoyl steht; oder
R² und R³ zusammengenommen eine 1,4-Butandiylgruppe bilden;
R⁴ für eine in der 4- und/oder 5-Stellung durch einen oder mehrere aus einer Gruppe (a) bestehend aus Nitro, Cyano, Halogen, C₁₋₄-Alkyl, C₁₋₄-Alkylsulfonyl und 2-Thienyl ausgewählte Reste substituierte 2- oder 3-Thienyl- oder Furylgruppe steht, mit der Maßgabe, daß eine 3-Thienyl- oder Furylgruppe nur in der 5-Stellung substituiert sein darf; oder
R⁴ für eine in der 5-Stellung und/oder entweder in der 4-Stellung oder in der 6-Stellung durch ein Mitglied der obigen Gruppe (a) substituierte 2-Pyridylgruppe steht; oder
R⁴ für eine in der 6-Stellung durch ein Mitglied der obigen Gruppe (a) substituierte 3-Pyridylgruppe steht; oder
R⁴ für eine in der 2-Stellung durch ein Mitglied der obigen Gruppe (a) substituierte 4-Pyridylgruppe steht; oder
R⁴ für eine Gruppe der Formel II: 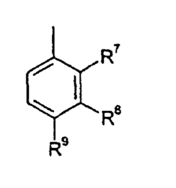 worin:
R⁷ für Wasserstoff steht und
R⁸ und R⁹ unabhängig voneinander unter Wasserstoff, Hydroxy-C₁₋₄-alkoxy, Nitro, Cyano, C₁₋₄-Fluoralkyl, C₁₋₄-Fluoralkoxy, Halogen, C₁₋₄-Alkyl, C₁₋₄-Alkanoyl, Phenyl und C₁₋₄-Alkylsulfonyl ausgewählt sind; oder
R⁸ und R⁹ zusammengenommen für C₁₋₃-Alkylendioxy stehen; steht; und
R¹⁰ und R¹¹ jeweils unabhängig voneinander für Wasserstoff oder C₁₋₄-Alkyl stehen, jedoch mit Ausnahme von 3-Cyano-4-phenyl-2,7,7-trimethyl-4,6,7,8-tetrahydro-5 (1H)-chinolon und 3-Ethanoyl-4-phenyl-2,7,7-trimethyl-4,6,7,8-tetrahydro-5(1H)-chinolon.

2. Verbindung nach Anspruch 1, in der entweder
R² für Methyl, Ethyl, Propyl, Isopropyl, Butyl, Isobutyl, sec.-Butyl, tert.-Butyl oder Trifluormethyl und
R³ für Wasserstoff, Cyano oder Ethanoyl steht oder
R² und R³ zusammengenommen eine 1,4-Butandiylgruppe bilden.

3. Verbindung nach Anspruch 2, in der R³ für Wasserstoff steht oder R² und R³ zusammengenommen eine 1,4-Butandiylgruppe bilden.

4. Verbindung nach Anspruch 3, in der R² für Trifluormethyl steht.

5. Verbindung nach einem der Ansprüche 1 bis 4, in der R⁴ für Brom-2-thienyl, 5-Brom-2-thienyl, 5-Methylsulfonyl-2-thienyl, 5-Methyl-2-thienyl, 5-(2-Thienyl)-2-thienyl, 4-Nitro-2-thienyl, 5-Nitro-2-thienyl, 4-Cyano-2-thienyl und 5-Nitro-3-thienyl oder eine Gruppe der Formel II, worin R⁸ unter Wasserstoff, Hydroxy, Methoxy, Nitro, Cyano, Trifluormethyl, Trifluormethoxy, Methyl, Ethyl, Isopropyl, Halogen und Ethanoyl und R⁹ unter Wasserstoff, Hydroxy, Methoxy, Nitro, Cyano, Trifluormethyl, Trifluormethoxy, Methyl, Ethyl, Isopropyl und Halogen ausgewählt ist, steht.

6. Verbindung nach einem der Ansprüche 1 bis 4, in der R⁴ für Phenyl, 3-Methoxyphenyl, 3-Nitrophenyl, 3-Cyanophenyl, 3-Trifluormethylphenyl, 3-Trifluormethyl-4-cyanophenyl, 4-Trifluormethylphenyl, 3-Trifluormethoxyphenyl, 3-Fluorphenyl, 3-Chlorphenyl, 3-Chlor-4-fluorphenyl, 3-Bromphenyl, 4-Fluorphenyl, 4-Chlorphenyl, 3-Brom-4-fluorphenyl, 3,4-Dichlorphenyl, 4-Methylphenyl, 3,4-Methylendioxyphenyl und 4-Nitro-2-thienyl steht.

7. Verbindung nach Anspruch 6, in der R⁴ für 3-Nitrophenyl oder 3-Cyanophenyl steht.

8. Verbindung nach einem der Ansprüche 1 bis 7, in der R¹⁰ und R¹¹ beide für Wasserstoff oder beide für Methyl stehen.

9. Verbindung nach Anspruch 1, in der entweder
R² für Methyl, Ethyl, Propyl, Isopropyl, Butyl, Isobutyl, sec.-Butyl, tert.-Butyl oder Trifluormethyl und
R³ für Wasserstoff steht oder
R² und R³ zusammengenommen eine 1,4-Butandiylgruppe bilden;
R⁴ für 3-Nitrophenyl oder 3-Cyanophenyl steht und
R¹⁰ und R¹¹ beide für Wasserstoff oder beide für stehen.

10. Verbindung nach Anspruch 1, die unter 2-Trifluormethyl-4-(3-nitrophenyl)-4,6,7,8-tetrahydro-5(1H)-chinolon und 2-Trifluormethyl-4-(3-cyanophenyl)-4,6,7,8-tetrahydro-5(1H)-chinolon ausgewählt ist.

11. Verfahren zur Herstellung einer Verbindung der Formel I oder eines pharmazeutisch unbedenklichen Salzes davon nach Anspruch 1, bei dem man
(a) zur Herstellung einer Verbindung der Formel I, worin R³ für Wasserstoff steht, eine entsprechende Carbonsäure der Formel III 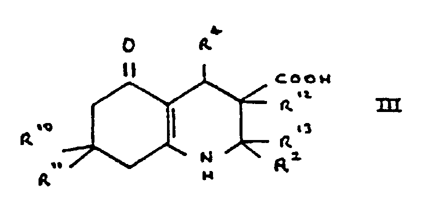 worin R¹² und R¹³ gemeinsam eine Bindung darstellen, decarboxyliert oder eine Carbonsäure der Formel III, worin R¹² für Wasserstoff und R¹³ für eine Hydroxylgruppe steht, decarboxyliert und dehydratisiert;
(b) ein ungesättigtes Keton der Formel IV 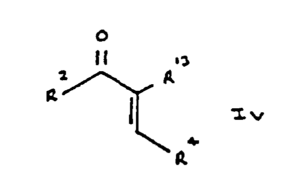 mit dem passenden 1,3-Cyclohexandion und Ammoniak oder einem Ammoniumsalz umsetzt;
(c) zur Herstellung einer Verbindung der Formel I, worin R² und R³ zusammengenommen eine 1,4-Butandiylgruppe bilden, ein Acridindion der Formel VI 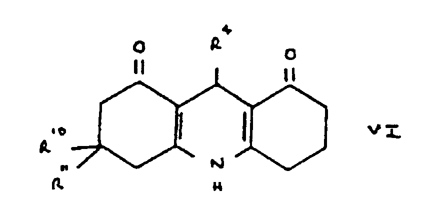 mit einem Reduktionsmittel umsetzt;
(d) zur Herstellung einer Verbindung der Formel I, worin R² und R³ zusammengenommen eine 1,4-Butandiylgruppe bilden, ein Keton der Formel VII 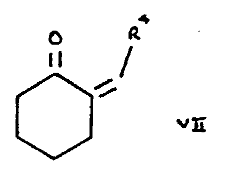 mit dem passenden 1,3-Cyclohexandion und Ammoniak oder einem Ammoniumsalz umsetzt;
(e) ein Endion der Formel XII 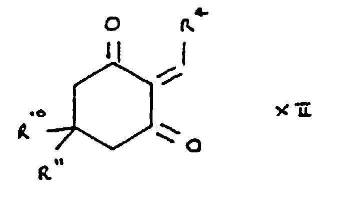 in Gegenwart von Ammoniak oder einem Ammoniumsalz mit einem Enamin der Formel XIII 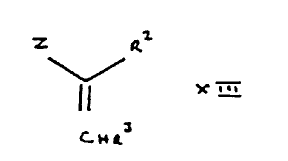 worin Z für eine Iminogruppe steht, umsetzt;
(f) zur Herstellung einer Verbindung der Formel I, worin R³ für Ethanoyl oder Cyano steht, eine Verbindung der Formel XV 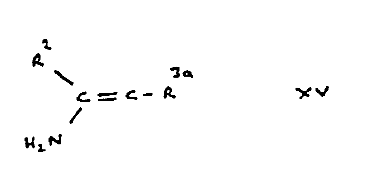 worin R^{3a} für Ethanoyl oder Cyano steht, mit dem passenden 1,3-Cyclohexandion und dem passenden Aldehyd der Formel R⁴CHO umsetzt,
wobei man für den Fall, daß eine optisch aktive Verbindung der Formel I gewünscht ist, die Verbindung der Formel I unter Verwendung optisch aktiver Edukte herstellt oder eine racemische Form der Verbindung der Formel I trennt und
für den Fall, daß ein pharmazeutisch unbedenkliches Salz gewünscht ist, eine Verbindung der Formel I mit einer geeigneten Säure oder Base, die ein physiologisch unbedenkliches Gegenion ergibt, umsetzt; und
R², R³, R⁴, R¹¹ und R¹² die in Anspruch 1 angegebenen Bedeutungen besitzen.

12. Pharmazeutische Zusammensetzung, die eine Verbindung der Formel I oder ein pharmazeutisch unbedenkliches Salz davon nach Anspruch 1 und ein pharmazeutisch unbedenkliches Verdünnungsmittel oder einen pharmazeutisch unbedenklichen Träger enthält.

13. Verwendung einer Verbindung der Formel I oder eines pharmazeutisch unbedenklichen Salzes davon nach Anspruch 1 bei der Herstellung eines Arzneimittels zur Behandlung von Harninkontinenz.

14. Verbindung der allgemeinen Formel III 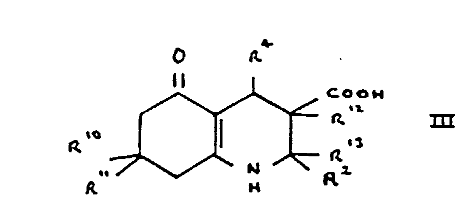 worin entweder R¹² und R¹³ gemeinsam eine Bindung darstellen oder R¹² für Wasserstoff und R¹³ für eine Hydroxylgruppe steht und R², R⁴, R¹⁰ und R¹¹ die in Anspruch 1 angegebenen Bedeutungen besitzen.

## Revendications

1. Composé de formule I : 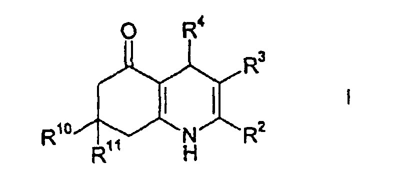 ou sel pharmaceutiquement acceptable de celui-ci, dans laquelle soit
R² est un hydrogène, un alkyle en C₁₋₆ ou un fluoroalkyle en C₁₋₄ ; et
R³ est un hydrogène, un cyano, un alkyle en C₁₋₆, un fluoroalkyle en C₁₋₆ ou un éthanoyle ; soit
R² et R³ forment, lorsqu'ils sont pris conjointement, un 1,4-butanediyle ;
R⁴ est un 2- ou 3-thiényle ou furyle substitué en position(s) 4 et/ou 5 par un radical ou des radicaux choisi(s) indépendamment parmi un groupe (a) constitué d'un nitro, d'un cyano, d'un halogéno, d'un alkyle en C₁₋₄, d'un C₁₋₄-alkylsulphonyle et d'un 2-thiényle, à condition qu'un groupe 3-thiényle ou furyle ne puisse être substitué qu'en position 5 ; ou
R⁴ est un 2-pyridyle substitué en position 5 et/ou soit en position 4 soit en position 6 par un membre du groupe (a) ci-dessus ; ou
R⁴ est un 3-pyridyle substitué en position 6 par un membre du groupe (a) ci-dessus ; ou
R⁴ est un 4-pyridyle substitué en position 2 par un membre du groupe (a) ci-dessus ; ou
R⁴ est un groupe de formule II : 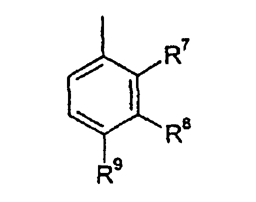 où :
R⁷ est un hydrogène ; et
R⁸ et R⁹ sont choisis indépendamment parmi un hydrogène, un hydroxy- C₁₋₄-alcoxy, un nitro, un cyano, un fluoroalkyle en C₁₋₄, un fluoroalcoxy en
C₁₋₄, un halogéno, un alkyle en C₁₋₄, un alcanoyle un
C₁₋₄, un phényle et un C₁₋₄-alkylsulphonyle ; ou
R⁸ et R⁹, pris conjointement, sont un C₁₋₃-alkylènedioxy ; et
R¹⁰ et R¹¹ sont chacun indépendamment un hydrogène ou un alkyle en C₁₋₄, mais à l'exclusion de la 3-cyano-4-phényl-2,7,7-triméthyl-4,6,7,8-tétrahydro-5(1H)-quinolone et de la 3-éthanoyl-4-phényl-2,7,7-triméthyle-4,6,7,8-tétrahydro-5 (1H)-quinolone.

2. Composé selon la revendication 1, dans lequel soit
R² est un méthyle, un éthyle, un propyle, un isopropyle, un butyle, un isobutyle, un sec-butyle, un tert-butyle ou un trifluorométhyle ; et
R³ est un hydrogène, un cyano, un éthanoyle ; soit
R² et R³ forment, lorsqu'ils sont pris conjointement, un 1,4-butanediyle.

3. Composé selon la revendication 2, dans lequel R³ est un hydrogène ; ou R² et R³ forment, lorsqu'ils sont pris conjointement, un 1,4-butanediyle.

4. Composé selon la revendication 3, dans lequel R² est un trifluorométhyle.

5. Composé selon l'une quelconque des revendications 1 à 4, dans lequel R⁴ est un bromo-2-thiényle, un 5-bromo-2-thiényle, un 5-méthylsulphonyl-2-thiényle, un 5-méthyl-2-thiényle, un 5-(2-thiényl)-2-thiényle, un 4-nitro-2-thiényle, un 5-nitro-2-thiényle, un 4-cyano-2-thiényle, et un 5-nitro-3-thiényle, ou est un groupe de formule II dans laquelle R⁸ est choisi parmi un hydrogène, un hydroxy, un méthoxy, un nitro, un cyano, un trifluorométhyle, un trifluorométhoxy, un méthyle, un éthyle, un isopropyle, un halogéno et un éthanoyle, et R⁹ est choisi parmi un hydrogène, un hydroxy, un méthoxy, un nitro, un cyano, un trifluorométhyle, un trifluorométhoxy, un méthyle, un éthyle, un isopropyle et un halogéno.

6. Composé selon l'une quelconque des revendications 1 à 4, dans lequel R⁴ est un phényle, 3-méthoxyphényle, un 3-nitrophényle, un 3-cyanophényle, un 3-trifluorométhylphényle, un 3-trifluorométhyle-4-cyanophényle, un 4-trifluorométhylphényle, un 3-trifluorométhoxyphényle, un 3-fluorophényle, un 3-chlorophényle, un 3-chloro-4-fluorophényle, un 3-bromophényle, un 4-fluorophényle, un 4-chlorophényle, un 3-bromo-4-fluorophényle, un 3,4-dichlorophényle, un 4-méthylphényle, un 3,4-méthylènedioxyphényle, et un 4-nitro-2-thiényle.

7. Composé selon la revendication 6, dans lequel R⁴ est un 3-nitrophényle ou un 3-cyanophényle.

8. Composé selon l'une quelconque des revendications 1 à 7, dans lequel R¹⁰ et R¹¹ sont tous les deux un hydrogène ou tous les deux un méthyle.

9. Composé selon la revendication 1, dans lequel soit
R² est un méthyle, un éthyle, un propyle, un isopropyle, un butyle, un isobutyle, un sec-butyle, un tert-butyle ou un trifluorométhyle ; et
R³ est un hydrogène ; soit
R² et R³ forment, lorsqu'ils sont pris conjointement, un 1,4-butanediyle ;
R⁴ est un 3-nitrophényle ou un 3-cyanophényle ; et
R¹⁰ et R¹¹ sont tous les deux un hydrogène.

10. Composé selon la revendication 1, qui est choisi parmi la 2-trifluorométhyl-4-(3-nitrophényl)-4,6,7,8-tétrahydro-5(1H)-quinolone et la 2-trifluorométhyl-4-(3-cyanophényl)-4,6,7,8-tétrahydro-5 (1H)-quinolone.

11. Procédé de préparation d'un composé de formule I ou d'un sel pharmaceutiquement acceptable de celui-ci tel que défini à la revendication 1, comprenant
(a) pour un composé de formule I dans laquelle R³ est un hydrogène, la décarboxylation d'un acide carboxylique correspondant de formule III 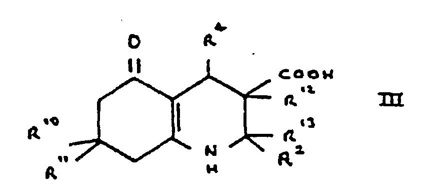 dans laquelle R¹² et R¹³ représentent conjointement une liaison, ou la décarboxylation et la déshydratation d'un acide carboxylique de formule III dans laquelle R¹² est un hydrogène et R¹³ est un groupe hydroxy,
(b) la réaction d'une cétone insaturée de formule IV 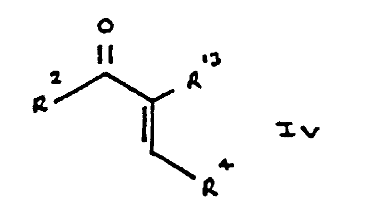 avec la 1,3-cyclohexanedione appropriée et de l'ammoniac ou un sel d'ammonium,
(c) pour un composé de formule I dans laquelle R² et R³ forment, lorsqu'ils sont pris conjointement, un 1,4-butanediyle, la réaction d'une acridinedione de formule VI 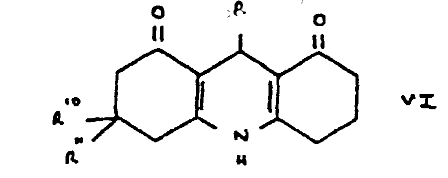 avec un réducteur,
(d) pour un composé de formule I dans laquelle R² et R³ forment, lorsqu'ils sont pris conjointement, un 1,4-butanediyle, la réaction d'une cétone de formule VII 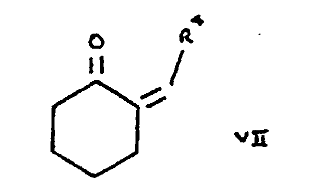 avec la 1,3-cyclohexanedione appropriée et de l'ammoniac ou un sel d'ammonium,
(e) la réaction d'une ènedione de formule XII 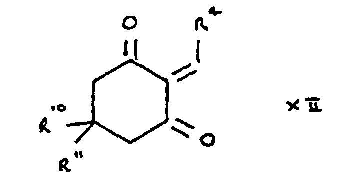 avec une énamine de formule XIII 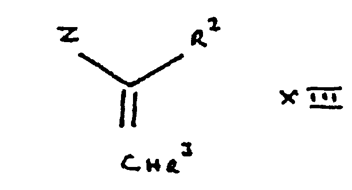 dans laquelle Z est un groupe imino, en présence d'ammoniac ou d'un sel d'ammonium,
(f) pour un composé de formule I dans laquelle R³ est un éthanoyl ou un cyano, la réaction d'un composé de formule XV 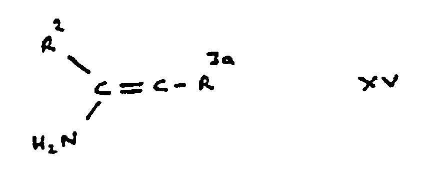 dans laquelle R^{3a} est un éthanoyle ou un cyano, avec la 1,3-cyclohexanedione appropriée et l'aldéhyde approprié de formule R⁴CHO,
où, si un composé optiquement actif de formule I est souhaité, le composé de formule I est préparé en utilisant une substance de départ optiquement active ou une forme racémique du composé de formule I est dédoublée, et
si un sel pharmaceutiquement acceptable est souhaité, un composé de formule I est mis à réagir avec un acide ou une base approprié(e) en donnant lieu à un contre-ion physiologiquement acceptable ; et
où R², R³, R⁴, R¹¹ et R¹² ont les significations indiquées à la revendication I.

12. Composition pharmaceutique comprenant un composé de formule I ou un seul pharmaceutiquement acceptable de celui-ci, tel que défini à la revendication 1, et un diluant ou support pharmaceutiquement acceptable.

13. Utilisation d'un composé de formule I ou d'un sel pharmaceutiquement acceptable de celui-ci tel que défini à la revendication I, pour l'élaboration d'un médicament destiné au traitement de l'incontinence urinaire.

14. Composé de formule générale III 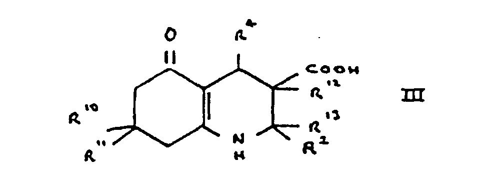 dans laquelle soit R¹² et R¹³ représentent conjointement une liaison, soit R¹² est un hydrogène et R¹³ est un groupe hydroxy, et R², R⁴, R¹⁰ et R¹¹ sont tels que définis à la revendication 1.
